# EUROPEAN PATENT APPLICATION

(11) **EP 4 299 721 A2**
(43) Date of publication of application: **03.01.2024**
(21) Application number: 23208039.0
(22) Date of filing: 08.05.2014
(51) Int. Cl.: C12N 5/071

(54) **ORGANOIDS COMPRISING ISOLATED RENAL CELLS AND USES THEREOF**

(30) Priority: 08.05.2013 US 201361855146 P; 09.05.2013 US 201361855152 P
(62) Divisional of application: 14730323.4
(71) Applicant: ProKidney, Grand Cayman KY1-9006 (KY)
(72) Inventor: BASU, Joydeep, Winston-Salem, NC 27103 (US); BRUCE, Andrew, Winston-Salem, NC 27103 (US); KELLEY, Rusty, Winston-Salem, NC 27103 (US); GUTHRIE, Kelly, Winston-Salem, NC 27103 (US)
(74) Representative: Mewburn Ellis LLP

(57) **Abstract**

Described herein are organoids comprising admixtures of selected bioactive primary renal cells and a bioactive cell population, e.g., an endothelial cell population, e.g. HUVEC cells, and methods of treating a subject in need thereof with such organoids. Further, the isolated renal cells, which may include tubular and erythropoietin (EPO)-producing kidney cell populations, and/or the endothelial cell populations may be of autologous, syngeneic, allogeneic or xenogeneic origin, or any combination thereof. Further provided are methods of treating a subject in need with the organoids.

## Description

### Field of the Invention

The invention is directed to admixtures of selected bioactive primary renal cells and further bioactive cell populations, and methods of treating a subject in need thereof. The invention is further directed to organoids comprising isolated renal cells, including tubular and erythropoietin (EPO)-producing kidney cell populations, and methods of treating a subject in need with the organoids.

### Background of the Invention

Chronic Kidney Disease (CKD) affects over 19M people in the United States and is frequently a consequence of metabolic disorders involving obesity, diabetes, and hypertension. Examination of the data reveals that the rate of increase is due to the development of renal failure secondary to hypertension and non-insulin dependent diabetes mellitus (NIDDM) (United States Renal Data System: Costs of CKD and ESRD. ed. Bethesda, MD, National Institutes of Health, National Institute of Diabetes and Digestive and Kidney Diseases, 2007 pp 223-238) - two diseases that are also on the rise worldwide. Obesity, hypertension, and poor glycemic control have all been shown to be independent risk factors for kidney damage, causing glomerular and tubular lesions and leading to proteinuria and other systemically-detectable alterations in renal filtration function (Aboushwareb, et al., World J Urol, 26: 295-300, 2008; Amann, K. et al., Nephrol Dial Transplant, 13: 1958-66, 1998). CKD patients in stages 1-3 of progression are managed by lifestyle changes and pharmacological interventions aimed at controlling the underlying disease state(s), while patients in stages 4-5 are managed by dialysis and a drug regimen that typically includes anti-hypertensive agents, erythropoiesis stimulating agents (ESAs), iron and vitamin D supplementation. According to the United States Renal Data Service (USRDS), the average end-stage renal disease (ESRD) patient expends >$600 per month on injectable erythropoiesis-stimulating agents (ESAs), Vitamin D supplements, and iron supplements (United States Renal Data System: Costs of CKD and ESRD. ed. Bethesda, MD, National Institutes of Health, National Institute of Diabetes and Digestive and Kidney Diseases, 2007 pp 223-238). When paired with the annual average cost of dialysis ($65,405), the healthcare cost for maintenance of a single patient rises to >$72,000/yr (United States Renal Data System: Costs of CKD and ESRO. ed. Bethesda, MD, National Institutes of Health, National Institute of Diabetes and Digestive and Kidney Diseases, 2007 pp 223-238) - a number that reflects only standard procedural costs and does not include treatment of other complications, emergency procedures, or ancillary procedures such as the placement of vascular grafts for dialysis access. Combined medicare costs for CKD and ESRD in 2005 totaled $628 - representing 19% of all medicare spending for that year (United States Renal Data System: Costs of CKD and ESRD. ed. Bethesda, MD, National Institutes of Health, National Institute of Diabetes and Digestive and Kidney Diseases, 2007 pp 223-238). Kidney transplantation is an effective option for stage 4-5 patients as a pre-emptive measure to avoid dialysis or when dialysis is no longer sufficient to manage the disease state, but the number of stage 5 CKD patients in the US (>400,000) who could benefit from whole kidney transplant far exceeds the number of suitable donor kidneys available in any given year (~16,000) (Powe, NR et al., Am J Kidney Dis, 53; S37-45, 2003). Thus, new treatment paradigms are needed to delay or reduce dependency on dialysis and to fill the void left by the shortage of donor kidneys.

Progressive renal disease results from a combination of the initial disease injury (e.g, hypertension), followed by a maladaptive renal response to that injury. Such a response includes the production of pro-inflammatory and pro-fibrotic cytokines and growth factors. Therefore, one strategy to slow CKD progression is to ameliorate the inflammatory and fibrotic response as well as mitigate or reverse renal degeneration through the repair and/or regeneration of renal tissue.

Chronic renal failure is prevalent in humans as well as some domesticated animals. Patients with renal failure experience not only the loss of kidney function (uremia), but also develop anemia due to the inability of the bone marrow to produce a sufficient number of red blood cells (RBCs) via erythropoiesis. Erythroid homeostasis is dependent on both the production of erythropoietin (EPO) by specialized interstitial fibroblasts that reside in the kidney and the ability of targeted erythroid progenitors in the bone marrow to respond to EPO and manufacture more RBCs. The anemia of renal failure is due to both reduced production of EPO in the kidney and the negative effects of uremic factors on the actions of EPQ in the bone marrow,

To date, clinical approaches to the treatment of chronic renal failure involve dialysis and kidney transplantation for restoration of renal filtration and urine production, and the systemic delivery of recombinant EPO or EPO analogs to restore erythroid mass. Dialysis offers survival benefit to patients in mid-to-late stage renal failure, but causes significant quality-of-life issues. Kidney transplant is a highly desired (and often the only) option for patients in the later stages of renal failure, but the supply of high-quality donor kidneys does not meet the demand for the renal failure population. Bolus dosing with recombinant EPO to treat anemia has now been associated with serious downstream health risks, leading to black box warnings from the FDA for the drug, and necessitating further investigation into alternative treatments to restore erythroid homeostasis in this population. Preclinical investigations have examined in vivo efficacy and safety of EPO-producing cells that have been generated via gene therapy approaches. These studies have shown that it is possible to transiently stimulate erythropoiesis and RBC number by in vivo delivery of epo-producing cells. However, to date, none of these approaches have offered regulated erythroid homeostasis or long-term in vivo functionality. Consequently, HCT and RBC number are often increased beyond normal values, leading to polycythemia vera and other complications. Delivery of EPO-producing cells that are therapeutically-relevant and provide advantages over delivery of recombinant EPO must not only increase HCT, but should restore erythroid homeostasis, with both positive and negative regulatory mechanisms intact. It is important to note that EPO-deficient anemias, while prevalent in patients with kidney disease, can also develop as a result of other disease states, including heart failure, multi-organ system failure, and other chronic diseases.

Regenerative medicine technologies provide next-generation therapeutic options for chronic kidney disease (CKD). Presnell et al. WO/2010/056328 and Ilagan et al. PCT/US2011/036347 describe isolated bioactive renal cells, including tubular and erythropoietin (EPO)-producing kidney cell populations, and methods of isolating and culturing the same, as well as methods of treating a subject in need with the cell populations.

There is a need for improved and more targeted regenerative medicine therapeutic options for subjects in need.

### SUMMARY OF THE INVENTION

Provided herein are organoids, methods for their preparation and use.

Organoids as described herein provide a therapeutic benefit to a subject in need without the use of a scaffold.

In one aspect there is provided a method of forming an organoid comprising a heterogeneous renal cell population and a bioactive cell population. In one emodiment, the metheod comprises culturing the heterogenerous renal cell population and a bioactive cell population in a culture system selected from the group consisting of i) 2D culture; ii) 3D culture: COL(I) gel; iii) 3D culture: Matrigel; iv) 3D culture: spinners, followed by COL(I)/Matrigel; and v) 3D culture: COL(IV) gel. In some embodiments, the heterogeneous renal cell population comprises a bioactive renal cell population. In certain embodiments, the heterogeneous renal cell population comprises a B2 cell population comprising an enriched population of tubular cells, and wherein the heterogeneous renal cell population is depleted of a B1 cell population and/or a B5 cell population or combination thereof. In some embodiments, the heterogeneous renal cell population comprises a cell population selected from B2, B2/B3, B2/B4, and B2/B3/B4. in embodiments, the heterogeneous renal cell population comprises erythropoetin (EPO)-producing cells.

In another embodiment, the bioactive cell population is an endothelial cell population. In certain embodiments, the endothelial cell population is a cell line. In some embodiments, the endothelial cell population is derived from human umbilical cord. In some embodiments, the bioactive cell population comprise endothelial progenitor cells. In some embodiments, the bioactive cell population comprise mesenchymal stem cells. In some embodiments, the endothelial cell population is adult-sourced, in some embodiments, the cell populations are selected from xenogeneic, syngeneic, allogeneic, autologous and combinations thereof.

In another embodiment, the heterogeneous renal cell population and the bioactive cell population are cultured separately for a first time period, combined and cultured for a second time period, in certain embodiments, the renal cell population and bioactive cell population are combined at a ratio of 1:1. In most embodiments, the renal cell population and bioactive cell population are combined, *e.g*., suspended, in growth medium. In some embodiments, the second time period is between 24 and 72 hours in length, preferably 24 hours.

In another aspect there is provided an organoid. In some embodiments, the organoids are made according to the methods described herein. In all embodiments, the organoids comprise a heterogeneous renal cell population and a bioactive cell population. In some embodiments, the bioactive cell population is an endothelial cell population. In some embodiments, the endothelial cell population is a cell line, In certain embodiments, the endothelial cell population comprises HUVEC cells.

In some embodiments, the heterogeneous renal cell population comprises a B2 cell population comprising an enriched population of tubular cells, and wherein the heterogeneous renal cell population is depleted of a B1 cell population. In certain embodiments, the heterogeneous renal cell population is further depleted of a B5 cell population. In select embodiments, the heterogeneous renal cell population comprises a cell population selected from B2, B2/B3, B2/B4, and B2/B3/B4. in some embodiments, the heterogeneous renal cell population comprises erythropoetin (EPO)-producing cells.

In a further aspect there is provided an injectable formulation comprising at least one organoid and a liquid medium. In one embodiment, the the liquid medium is selected from a cell growth medium, DPBS and combinations thereof. In another embodiment, the organoids are suspended in the liquid medium.

in a second embodiment, the injectable formulation comprises organoids and a temperature-sensitive cell-stabilizing biomaterial that maintains (i) a substantially solid state at about 8°C or below, and (ii) a substantially liquid state at about ambient temperature or above. In one other embodiment, the bioactive cells comprise renal cells, as described herein. In another embodiment, the bioactive cells are substantially uniformly dispersed throughout the volume of the cell-stabilizing biomaterial. In other embodiments, the biomaterial has a solid-to-liquid transitional state between about 8°C and about ambient temperature or above. In one embodiment, the substantially solid state is a gel state. In another embodiment, the cell-stabilizing biomaterial comprises a hydrogel. In one other embodiment, the hydrogel comprises gelatin. In other embodiments, the gelatin is present in the formulation at about 0.5% to about 1% (w/v). In one embodiment, the gelatin is present in the formulation at about 0.75% (w/v).

In an aspect there is provided a method of treating kidney disease in a subject in need comprising administering at least one organoid comprising a heterogeneous renal cell population and a bioactive cell population. In some embodiments, the bioactive cell population is an endothelial cell population, In certain embodiments, the endothelial cell population is a cell line. In one embodiment, the endothelial cell population comprises HUVEC cells.

In most embodiments, the heterogeneous renal cell population comprises a B2 cell population comprising an enriched population of tubular cells, and wherein the heterogeneous renal cell population is depleted of a B1 cell population. In select embodiments, the heterogeneous renal cell population is further depleted of a B5 cell population. In some embodiments, the heterogeneous renal cell population comprises a cell population selected from B2, B2/B3, B2/B4, and B2/B3/B4. In most embodiments, the heterogeneous renal cell population comprises erythropoetin (EPO)-producing cells.

In an embodiment, the method of treating kidney disease in a subject in need comprising administering an injectable formulation as described herein. In all embodiments, the subject is a mammal selected from dogs, cats, horses, rabbits, zoo animals, cows, pigs, sheep, and primates. In a specific embodiment, the mammal is a human. In all embodiments, the subject has a kidney disease. In embodiments, an improvement in any one of the following measures of anemia (Het, Hgb, RBC), inflammation (WBC), urine concentration (spGrav) and azotemia (BUN) is observed.

In another aspect, the use of an organoid in the preparation of a medicament for the treatment of kidney disease is provided.

### BRIEF DESCRIPTION OF THE DRAWINGS

**FIG, 1** depicts cell culture morphology of primary ZSF1 cells on fibronectin-coated plates. **A.** p0 unfractionated presort viewed at 5*x* magnification; **B.** p1 CD31⁺ viewed at 5*x* magnification; **C.** primary culture of unfractionated kidney cells at the end of passage 0 grown in 21%O₂ on fibronectin-treated flask in KGM; **D.** negative flow through (CD31⁻) from *Miltenyi* micro-beads selection,-and **E. 90%** CD31⁺ cells selected at the end of passage 1 grown on fibronectin coated flasks in EGM2 fully supplemented medium,
**FIG. 2** shows human cell culture morphology viewed at 5*x*. **A.** Unfractionated kidney cells at the end of p0 that were grown in 21%O₂ on TC-treated flask in KGM; **B.** Unfractionated kidney cells at the end of passage 0 grown exposed to 2%O₂ O/N on TC-treated flask in KGM; C. Unfractionated kidney cells at the end of passage 0 on fibronectin coated flasks in EGM2 fully supplemented medium; **D,** CD31⁺ positively selected cells at the end of passage 1 grown on fibronectin coated flasks in fully supplemented EGM2 media.
**FIG. 3** depicts FACS analysis showing percentage positive CD31 endothelial cells in selected samples during culture period. The unfractionated (UNFX) endothelial composition was <3% at p0 and was enriched ~ 15 fold at p1 when plated on fibronectin and cultured in EGM-2 media.
FIG. 3-1 shows Bigeneic Cre/IoxP reporters for lineage tracing studies.
   **A.** Six2-Cre x R26td Tomato Red cross traces epithelial cells: parietal, proximal and distal epithelial cells, but not interstitial or collecting duct. **B.** Unlabeled control. **C.** Six-2 positivity from p1 culture (3 day normoxic followed by 1 day hypoxic culture).
**FIG. 4** shows spinner flasks (A) and low bind plates on rotator (B) used for SRC organoid formation.
**FIG. 5** depicts phase imaging (*10x*) of **A.** rat and **B.** human SRC showing uniformity in size. **C.** Organoids expressing pan-cadherin phenotype (green), nuclear (blue) (*20x*),
**FIG. 6** shows human organoids cultured within a 3D collagen I/IV gel. **A.** Phase image showing low magnification of organoid tube formation (ringed in white). **B.** Phase image at higher magnification (ringed in white) along with the remnant organoids, **C.** GGT-1 phenotypic expression (green), nuclear (blue) **D,** CK18 expression (green), nuclear (blue) at 20x magnification.
**FIG. 7** depicts membrane dye labeled organoids. **A.** SRC only labeled with DiL (red) at x100 maginification. **B.** Organoid *plus*, SRC labeled with Oil (red), HuVEC labeled with DiO (green) at x100 maginification.
**FIG. 8** shows 3D tubulogenesis assay in Col I/IV gel of self-generated organoids *plus* in panels A, **B** ,and C showing the presence of both SRC population (red) and endothelial cells (green), When merged, populations appear yellow. Nuclear staining (blue) at 20x magnification.
**FIG. 9** depicts SPIO Rhodamine labeled organoid (red) prior to injection.
**FIG. 10** shows magnetic resonance imaging (MRI) of organoid retention post-implantation green= cells. **A.** 24hrs. **B.** 48hrs post-injection.
**FIG. 11** depicts prussian blue staining of implanted organoids showing cell retention and bio-distribution at low and high magnification. **A.** Implanted left kidney 24hrs post implant. **B.** implanted left kidney 48hrs post-implant.

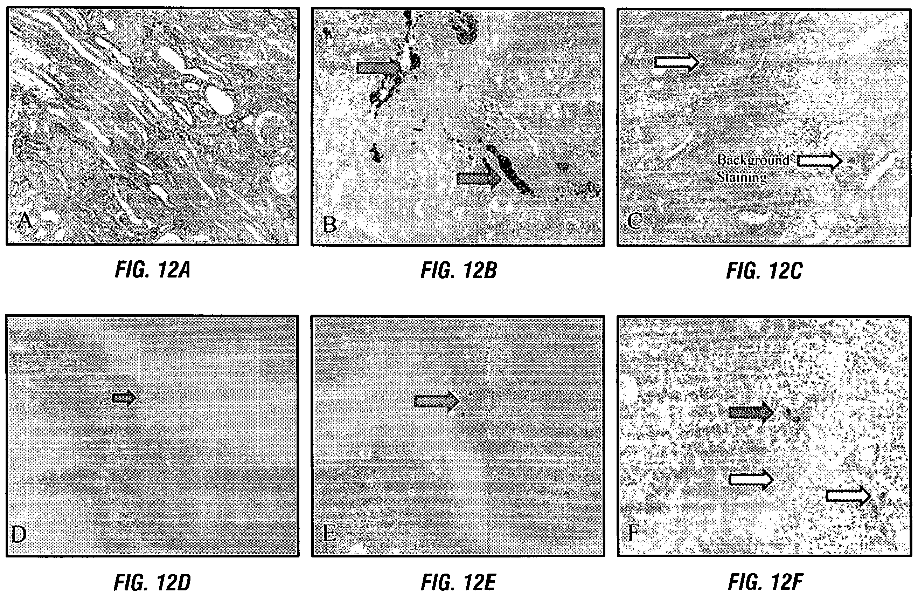

**FIG. 12** is a panel of photographs showing the HLA1 staining of human cells in a rat kidney that had been administered the organoids described herein. **A.** Normal human kidney; B. Human kidney cells in rat kidney. **C.** Untreated Nephrectomized rat kidney. **D.** NKO treated (low power micoscopy). **E.** NKO treated (high power microscopy). **F.** A second NKO treated animal. Panels A and **B** demonstrate staining (brown) of normal human kidney tissue as well as human kidney cells (green arrows) acutely delivered to a rodent kidney. Background staining is present in end of study non-treated diseased rat kidneys presumably due to "sticky" nature of proteinaceous casts and damaged tubules as identified in panels C and F by yellow arrows. This staining is typically lighter in color but is sometimes dark and of size smaller than cells. Panels D, E and F demonstrate lower magnification utilized for screening to identify dark staining cells, then higher magnification confirmation of HLA1 staining cells (green arrows).

The patent or application file contains at least one drawing in color. Copies of this patent or patent publication with color drawing(s) will be provided by the Office upon request and payment of the necessary fee.

### DETAILED DESCRIPTION OF THE INVENTION

Many cell types make up the nephron, the functional unit within the kidney parenchyma. The ability to isolate therapeutically bioactive renal cells from both normal and diseased animals and humans has been recently established ¹⁻³. The method used in these studies is dependent on the isolation of a mixture of renal cells that exist in a diseased tissue biopsy, using buoyant density gradient centrifugation.

The present invention, in one aspect, conerns the isolation, identification and expansion of selected individual cell populations of the various nephron compartments or niches, such that novel, multiple enriched cell types can be combined as selected admixtures. The novel selected admixtures of the present invention provide improved targeting of specific structural and functional deficiencies associated with the clinical and pathophysiologic basis of renal disease. The isolation and enrichment of multiple, individual cell types that make up the riephron and combined as selected admixtures enables improved targeted treatment of specific renal disease cohorts.

Cell types such as the vascular endothelium, tubular and collecting duct epithelium, interstitial cells, glomerular cells, mesenchymal stem cells, etc., can be isolated, identified and expanded *ex-vivo.* While each cell type may require a unique method and media formulation for sub-culture, they can be added back in selective combinations or admixtures as organoid clusters to provide enhanced delivery and improved treatment for an underlying renal tissue/ cell deficiency associated with a specific acute and/or chronic kidney disease patient syndrome/cohort ⁸.

The present invention contemplates methods of treating renal impairment associated with diseases of the vasculature (*e.g*., hypertension, microangiopathic anemias) using a defined ratio of renal tubular epithelial to endothelial cells. The ability to isolate, characterize and expand resident renal endothelial cells using selective culture systems and magnetic sorting allows for enrichment of a previously characterized selective regenerative renal epithelial cell (SRC) population ^{2,4} with a specific percentage of purified renal endothelial cells (SRC+). The SRC+ cell populations of the present invention are comprised of selected renal cells (SRCs or BRCs), as previously described and also desribed herein, and further bioactive cell populations, including but not limted to, endothelial cells, endothelial progenitors, mesenchymal stem cells, and/or adipose-derived progenitors. In one embodiment, the further bioactive cell populations are sourced from renal sources. In other embodiments, the further bioactive cell populations are sourced from non-renal sources.

The present invention, in one aspect, is directed to organoids comprising and/or formed from heterogenous mixtures or fractions of selected or bioactive renal cells (SRCs or BRCs) or SRC+ cell poulations, methods of isolating and culturing the same, as well as methods of treatment using the organoids as described herein. Directed delivery of SRC or SRC+ populations to the kidney as organoids is expected to improve cell retention, thereby leading to improved overall therapeutic outcomes. The present invention is also directed to methods of treatment using SRC+ cell populations.

### Definitions

Unless defined otherwise, technical and scientific terms used herein have the same meaning as commonly understood by one of ordinary skill in the art to which this invention belongs. Principles of Tissue Engineering, 3rd Ed. (Edited by R Lanza, R Langer, & J Vacanti), 2007 provides one skilled in the art with a general guide to many of the terms used in the present application. One skilled in the art will recognize many methods and materials similar or equivalent to those described herein, which could be used in the practice of the present invention, Indeed, the present invention is in no way limited to the methods and materials described.

The term "cell population" as used herein refers to a number of cells obtained by isolation directly from a suitable tissue source, usually from a mammal. The isolated cell population may be subsequently cultured *in vitro.* Those of ordinary skill in the art will appreciate that various methods for isolating and culturing cell populations for use with the present invention and various numbers of cells in a cell population that are suitable for use in the present invention. A cell population may be an unfractionated, heterogeneous cell population derived from the kidney. For example, a heterogeneous cell population may be isolated from a kidney biopsy or from whole kidney tissue. Alternatively, the heterogeneous cell population may be derived from *in vitro* cultures of mammalian cells, established from kidney biopsies or whole kidney tissue. An unfractionated heterogeneous cell population may also be referred to as a non-enriched cell population.

The term "native kidney" shall mean the kidney of a living subject. The subject may be healthy or un-healthy. An unhealthy subject may have a kidney disease.

The term "regenerative effect" shall mean an effect which provides a benefit to a native kidney. The effect may include, without limitation, a reduction in the degree of injury to a native kidney or an improvement in, restoration of, or stabilization of a native kidney function. Renal injury may be in the form of fibrosis, inflammation, glomerular hypertrophy, etc. and related to kidney disease in the subject.

The term "regenerative potential" or "potential regenerative bioactivity" as used herein refers to the potential of the organoids comprising bioactive cell preparations and/or admixtures described herein to provide a regenerative effect.

The term "admixture" as used herein refers to a combination of two or more isolated, enriched cell populations derived from an unfractionated, heterogeneous cell population. According to certain embodiments, the cell populations of the present invention are renal cell populations.

An "enriched" cell population or preparation refers to a cell population derived from a starting kidney cell population (*e.g*., an unfractionated, heterogeneous cell population) that contains a greater percentage of a specific cell type than the percentage of that cell type in the starting population. For example, a starting kidney cell population can be enriched for a first, a second, a third, a fourth, a fifth, and so on, cell population of interest. As used herein, the terms "cell population", "cell preparation" and "cell prototype" are used interchangeably,

In one aspect, the term "enriched" cell population as used herein refers to a cell population derived from a starting kidney cell population (e.g., a cell suspension from a kidney biopsy or cultured mammalian kidney cells) that contains a percentage of cells capable of producing EPO that is greater than the percentage of cells capable of producing EPO in the starting population. For example, the term "B4" is a cell population derived from a starting kidney cell population that contains a greater percentage of EPO-producing cells, glomerular cells, and vascular cells as compared to the starting population. The cell populations of the present invention may be enriched for one or more cell types and depleted of one or more other cell types. For example, an enriched EPO-producing cell population may be enriched for interstitial fibroblasts and depleted of tubular cells and collecting duct epithelial cells relative to the interstitial fibroblasts and tubular cells in a non-enriched cell population, i.e. the starting cell population from which the enriched cell population is derived. In all embodiments citing EPO-enriched or "B4" populations, the enriched cell populations are heterogeneous populations of cells containing cells that can produce EPO in an oxygen-regulated manner, as demonstrated by oxygen-tunable EPO expression from the endogenous native EPO gene.

In another aspect, an enriched cell population, which contains a greater percentage of a specific cell type, *e.g*., vascular, glomerular, or endocrine cells, than the percentage of that cell type in the starting population, may also lack or be deficient in one or more specific cell types, *e.g*., vascular, glomerular, or endocrine cells, as compared to a starting kidney cell population derived from a healthy individual or subject. For example, the term "B4'," or B4 prime," in one aspect, is a cell population derived from a starting kidney cell population that lacks or is deficient in one or more cell types, *e.g*., vascular, glomerular or endocrine, depending on the disease state of the starting specimen, as compared to a healthy individual. In one embodiment, the B4' cell population is derived from a subject having chronic kidney disease. In one embodiment, the B4' cell population is derived from a subject having focal segmental glomerulosclerosis (FSGS). In another embodiment, the B4' cell population is derived from a subject having autoimmune glomerulonephritis, in another aspect, B4' is a cell population derived from a starting cell population including all cell types, *e.g*., vascular, glomerular, or endocrine cells, which is later depleted of or made deficient in one or more cell types, *e.g*., vascular, glomerular, or endocrine cells. In yet another aspect, B4' is a cell population derived from a starting cell population including all cell types, *e.g*., vascular, glomerular, or endocrine cells, in which one or more specific cell types *e.g*., vascular, glomerular, or endocrine cells, is later enriched. For example, in one embodiment, a B4' cell population may be enriched for vascular cells but depleted of glomerular and/or endocrine cells, in another embodiment, a B4' cell population may be enriched for glomerular cells but depleted of vascular and/or endocrine cells. In another embodiment, a B4' cell population may be enriched for endocrine cells but depleted of vascular and/or glomerular cells. In another embodiment, a B4' cell population may be enriched for vascular and endocrine cells but depleted of glomerular cells, In preferred embodiments, the B4' cell population, alone or admixed with another enriched cell population, *e.g*., B2 and/or B3, retains therapeutic properties. A B4' cell population, for example, is described herein in the Examples, e.g., Examples 7-9.

In another aspect, an enriched cell population may also refer to a cell population derived from a starting kidney cell population as discussed above that contains a percentage of cells expressing one or more vascular, glomerular and proximal tubular markers with some EPO-producing cells that is greater than the percentage of cells expressing one or more vascular, glomerular and proximal tubular markers with some EPO-producing cells in the starting population. For example, the term "B3" refers to a cell population derived from a starting kidney cell population that contains a greater percentage of proximal tubular cells as well as vascular and glomerular cells as compared to the starting population. In one embodiment, the B3 cell population contains a greater percentage of proximal tubular cells as compared to the starting population but a lesser percentage of proximal tubular cells as compared to the B2 cell population. In another embodiment, the B3 cell population contains a greater percentage of vascular and glomerular cells markers with some EPO-producing cells as compared to the starting population but a lesser percentage of vascular and glomerular cells markers with some EPO-producing cells as compared to the B4 cell population.

In another aspect, an enriched cell population may also refer to a cell population derived from a starting kidney cell population as discussed above that contains a percentage of cells expressing one or more tubular cell markers that is greater than the percentage of cells expressing one or more tubular cell markers in the starting population. For example, the term "B2" refers to a cell population derived from a starting kidney cell population that contains a greater percentage of tubular cells as compared to the starting population. In addition, a cell population enriched for cells that express one or more tubular cell markers (or "B2") may contain some epithelial cells from the collecting duct system. Although the cell population enriched for cells that express one or more tubular cell markers (or "B2") is relatively depleted of EPO-producing cells, glomerular cells, and vascular cells, the enriched population may contain a smaller percentage of these cells (EPO- producing, glomerular, and vascular) in comparison to the starting population. In general, a heterogeneous cell population is depleted of one or more cell types such that the depleted cell population contains a lesser proportion of the cell type(s) relative to the proportion of the cell type(s) contained in the heterogeneous cell population prior to depletion. The cell types that may be depleted are any type of kidney cell. For example, in certain embodiments, the cell types that may be depleted include cells with large granularity of the collecting duct and tubular system having a density of < about 1.045 g/ml, referred to as "81". In certain other embodiments, the cell types that may be depleted include debris and small cells of low granularity and viabilty having a density of > about 1.095 g/ml, referred to as "85". In some embodiments, the cell population enriched for tubular cells is relatively depleted of all of the following: "B1", "B5", oxygen-tunable EPO-expressing cells, glomerular cells, and vascular cells.

The term "hypoxic" culture conditions as used herein refers to culture conditions in which cells are subjected to a reduction in available oxygen levels in the culture system relative to standard culture conditions in which cells are cultured at atmospheric oxygen levels (about 21%). Non-hypoxic conditions are referred to herein as normal or normoxic culture conditions.

The term "oxygen-tunable" as used herein refers to the ability of cells to modulate gene expression (up or down) based on the amount of oxygen available to the cells, "Hypoxia-inducible" refers to the upregulation of gene expression in response to a reduction in oxygen tension (regardless of the pre-induction or starting oxygen tension).

The term "spheroid" refers to an aggregate or assembly of cells cultured to allow 3D growth as opposed to growth as a monolayer, It is noted that the term "spheroid" does not imply that the aggregate is a geometric sphere. The aggregate may be highly organized with a well defined morphology or it may be an unorganized mass; it may include a single cell type or more than one cell type. The cells may be primary isolates, or a permanent cell line, or a combination of the two. Included in this definition are organoids and organotypic cultures.

The term "organoid" as used herein refers to a heterogeneous 3D agglomeration of cells that recapitulates aspects of cellular self-organization, architecture and signaling interactions present in the native organ. The term "organoid" includes spheroids or cell clusters formed from suspension cell cultures.

The term "biomaterial" as used here refers to a natural or synthetic biocompatible material that is suitable for introduction into living tissue. A natural biomaterial is a material that is made by a living system. Synthetic biomaterials are materials which are not made by a living system. The biomaterials disclosed herein may be a combination of natural and synthetic biocompatible materials. As used herein, biomaterials include, for example, polymeric matrices and scaffolds. Those of ordinary skill in the art will appreciate that the biomaterial(s) may be configured in various forms, for example, as liquid hydrogel suspensions, porous foam, and may comprise one or more natural or synthetic biocompatible materials.

The term "construct" refers to one or more cell populations deposited on or in a surface of a scaffold or matrix made up of one or more synthetic or naturally-occurring biocompatible materials. The one or more cell populations may be coated with, deposited on, embedded in, attached to, seeded, or entrapped in a biomaterial made up of one or more synthetic or naturally-occurring biocompatible polymers, proteins, or peptides. The one or more cell populations may be combined with a biomaterial or scaffold or matrix *in vitro or in vivo.* In general, the one or more biocompatible materials used to form the scaffold/biomaterial is selected to direct, facilitate, or permit the formation of multicellular, three-dimensional, organization of at least one of the cell populations deposited thereon. The one or more biomaterials used to generate the construct may also be selected to direct, facilitate, or permit dispersion and/or integration of the construct or cellular components of the construct with the endogenous host tissue, or to direct, facilitate, or permit the survival, engraftment, tolerance, or functional performance of the construct or cellular components of the construct.

The term "marker" or "biomarker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a particular type of cell. The marker may be a polypeptide expressed by the cell or an identifiable physical location on a chromosome, such as a gene, a restriction endonuclease recognition site or a nucleic acid encoding a polypeptide (e.g., an mRNA) expressed by the native cell. The marker may be an expressed region of a gene referred to as a "gene expression marker", or some segment of DNA with no known coding function. The biomarkers may be cell-derived, e.g., secreted, products.

The terms "differentially expressed gene," "differential gene expression" and their synonyms, which are used interchangeably, refer to a gene whose expression is activated to a higher or lower level in a first cell or cell population, relative to its expression in a second cell or cell population. The terms also include genes whose expression is activated to a higher or lower level at different stages over time during passage of the first or second cell in culture. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between the first cell and the second cell. Differential expression includes both quantitative, as well as qualitative, differences in the temporal or cellular expression pattern in a gene or its expression products among, for example, the first cell and the second cell. For the purpose of this invention, "differential gene expression" is considered to be present when there is a difference between the expression of a given gene in the first cell and the second cell. The differential expression of a marker may be in cells from a patient before administration of a cell population, admixture, or construct (the first cell) relative to expression in cells from the patient after administration (the second cell),

The terms "inhibit", "'down-regulate", "under-express" and "reduce" are used interchangeably and mean that the expression of a gene, or level of RNA molecules or equivalent RNA molecules encoding one or more proteins or protein subunits, or activity of one or more proteins or protein subunits, is reduced relative to one or more controls, such as, for example, one or more positive and/or negative controls. The under-expression may be in cells from a patient before administration of a cell population, admixture, or construct relative to cells from the patient after administration.

The term "up-regulate" or "over-express" is used to mean that the expression of a gene, or level of RNA molecules or equivalent RNA molecules encoding one or more proteins or protein subunits, or activity of one or more proteins or protein subunits, is elevated relative to one or more controls, such as, for example, one or more positive and/or negative controls. The over-expression may be in cells from a patient after administration of a cell population, admixture, or construct relative to cells from the patient before administration.

The term "anemia" as used herein refers to a deficit in red blood cell number and/or hemoglobin levels due to inadequate production of functional EPO protein by the EPO-producing cells of a subject, and/or inadequate release of EPO protein into systemic circulation, and/or the inability of erythroblasts in the bone marrow to respond to EPO protein. A subject with anemia is unable to maintain erythroid homeostasis. In general, anemia can occur with a decline or loss of kidney function (e.g., chronic renal failure), anemia associated with relative EPO deficiency, anemia associated with congestive heart failure, anemia associated with myelo-suppressive therapy such as chemotherapy or anti - viral therapy (e.g., AZT), anemia associated with non-myeloid cancers, anemia associated with viral infections such as HIV, and anemia of chronic diseases such as autoimmune diseases (e.g., rheumatoid arthritis), liver disease, and multi-organ system failure.

The term "EPO-deficiency" refers to any condition or disorder that is treatable with an erythropoietin receptor agonist (e.g., recombinant EPO or EPO analogs), including anemia.

The term "organ-related disease" as used herein refers to disorders associated with any stage or degree of acute or chronic organ failure that results in a loss of the organ's ability to perform its function.

The term "kidney disease" as used herein refers to disorders associated with any stage or degree of acute or chronic renal failure that results in a loss of the kidney's ability to perform the function of blood filtration and elimination of excess fluid, electrolytes, and wastes from the blood. Kidney disease also includes endocrine dysfunctions such as anemia (erythropoietin-deficiency), and mineral imbalance (Vitamin D deficiency). Kidney disease may originate in the kidney or may be secondary to a variety of conditions, including (but not limited to) heart failure, hypertension, diabetes, autoimmune disease, or liver disease. Kidney disease may be a condition of chronic renal failure that develops after an acute injury to the kidney. For example, injury to the kidney by ischemia and/or exposure to toxicants may cause acute renal failure; incomplete recovery after acute kidney injury may lead to the development of chronic renal failure.

The term "treatment" refers to both therapeutic treatment and prophylactic or preventative measures for kidney disease, anemia, EPO deficiency, tubular transport deficiency, or glomerular filtration deficiency wherein the object is to reverse, prevent or slow down (lessen) the targeted disorder. Those in need of treatment include those already having a kidney disease, anemia, EPO deficiency, tubular transport deficiency, or glomerular filtration deficiency as well as those prone to having a kidney disease, anemia, EPO deficiency, tubular transport deficiency, or glomerular filtration deficiency or those in whom the kidney disease, anemia, EPO deficiency, tubular transport deficiency, or glomerular filtration deficiency is to be prevented. The term "treatment" as used herein includes the stabilization and/or improvement of kidney function.

The term "subject" shall mean any single human subject, including a patient, eligible for treatment, who is experiencing or has experienced one or more signs, symptoms, or other indicators of a kidney disease, anemia, or EPO deficiency. Such subjects include without limitation subjects who are newly diagnosed or previously diagnosed and are now experiencing a recurrence or relapse, or are at risk for a kidney disease, anemia, or EPO deficiency, no matter the cause. The subject may have been previously treated for a kidney disease, anemia, or EPO deficiency, or not so treated.

The term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

The term "sample" or "patient sample" or "biological sample" shall generally mean any biological sample obtained from a subject or patient, body fluid, body tissue, cell line, tissue culture, or other source. The term includes tissue biopsies such as, for example, kidney biopsies. The term includes cultured cells such as, for example, cultured mammalian kidney cells. Methods for obtaining tissue biopsies and cultured cells from mammals are well known in the art. If the term "sample" is used alone, it shall still mean that the "sample" is a "biological sample" or "patient sample", *i.e*., the terms are used interchangeably.

The term "test sample" refers to a sample from a subject that has been treated by a method of the present invention. The test sample may originate from various sources in the mammalian subject including, without limitation, blood, semen, serum, urine, bone marrow, mucosa, tissue, etc.

The term "control" or "control sample" refers a negative or positive control in which a negative or positive result is expected to help correlate a result in the test sample. Controls that are suitable for the present invention include, without limitation, a sample known to exhibit indicators characteristic of normal erythroid homeostasis, a sample known to exhibit indicators characteristic of anemia, a sample obtained from a subject known not to be anemic, and a sample obtained from a subject known to be anemic. Additional controls suitable for use in the methods of the present invention include, without limitation, samples derived from subjects that have been treated with pharmacological agents known to modulate erythropoiesis (e.g., recombinant EPO or EPO analogs). In addition, the control may be a sample obtained from a subject prior to being treated by a method of the present invention. An additional suitable control may be a test sample obtained from a subject known to have any type or stage of kidney disease, and a sample from a subject known not to have any type or stage of kidney disease. A control may be a normal healthy matched control. Those of skill in the art will appreciate other controls suitable for use in the present invention.

"Regeneration prognosis", "regenerative prognosis", or "prognostic for regeneration" generally refers to a forecast or prediction of the probable regenerative course or outcome of the administration or implantation of a cell population, admixture or construct described herein. For a regeneration prognosis, the forecast or prediction may be informed by one or more of the following: improvement of a functional organ (*e.g.*, the kidney) after implantation or administration, development of a functional kidney after implantation or administration, development of improved kidney function or capacity after implantation or administration, and expression of certain markers by the native kidney following implantation or administration.

"Regenerated organ" refers to a native organ after implantation or administration of a cell population, admixture, or construct as described herein. The regenerated organ is characterized by various indicators including, without limitation, development of function or capacity in the native organ, improvement of function or capacity in the native organ, and the expression of certain markers in the native organ. Those of ordinary skill in the art will appreciate that other indicators may be suitable for characterizing a regenerated organ.

"Regenerated kidney" refers to a native kidney after implantation or administration of a cell population, admixture, or construct as described herein. The regenerated kidney is characterized by various indicators including, without limitation, development of function or capacity in the native kidney, improvement of function or capacity in the native kidney, and the expression of certain markers in the native kidney. Those of ordinary skill in the art will appreciate that other indicators may be suitable for characterizing a regenerated kidney,

### SRC+ cell populations

The present invention provides cel! populations comprising isolated, heterogeneous populations of kidney cells, enriched for specific bioactive components or cell types and/or depleted of specific inactive or undesired components or cell types and further bioactive cell populations, including but not limted to, endothelial cells, endothelial progenitors, mesenchymal stem cells, adipose-derived progenitors, for use in the treatment of acute or chronic kidney disease. The isolated, heterogeneous populations of kidney cells, enriched for specific bioactive components or cell types and/or depleted of specific inactive or undesired components or cell types may include any of the cell populations as described herein. In one embodiment, the further bioactive components, *e.g.,* endothelial cells, endothelial progenitors, mesenchymal stem cells, adipose-derived progenitors, are admixed with the isolated, heterogeneous populations of kidney cells, enriched for specific bioactive components or cell types and/or depleted of specific inactive or undesired components or cell types. The present invention, in another aspect, provides methods for preparing the SRC+ cell populations, as described herein. The further bioactive components, *e.g*., endothelial cells, endothelial progenitors, mesenchymal stem cells, adipose-derived progenitors, comprising the cell population, may be present in any percentage sufficient to improve a cell or tissue deficiency.

The cell populations of the invention may comprise one or more further bioactive components, for example but not limited to, endothelial cells, endothelial progenitors, mesenchymal stem cells, adipose-derived progenitors, comprising the cell population. In certain embodiments, the cell population comprises two further bioactive components, In certain embodiments, the cell population comprises three further bioactive components. In certain embodiments, the cell population comprises four further bioactive components. In certain embodiments, the cell population comprises five further bioactive components. In certain embodiments, the cell population comprises six further bioactive components. In certain embodiments, the cell population comprises seven further bioactive components, in certain embodiments, the cell population comprises eight further bioactive components, in certain embodiments, the cell population comprises nine further bioactive components. In certain embodiments, the cell population comprises ten further bioactive components.

In one embodiment, a further bioactive component is an epithelial cell. In one embodiment, the epithelial cell is a proximal tubular epithelial cell. In another embodiment, the epithelial cell is a distal tubular epithelial cell. In another embodiment, the epithelial cell is a parietal epithelial cell.

In another embodiment, a further bioactive component is an epithelial cell. In one embodiment, the epithelial cell is a venous endothelial cell. In one embodiment, the epithelial cell is an arterial endothelial cell. In one embodiment, the epithelial cell is a capillary endothelial cell. In one embodiment, the epithelial cell is a lymphatic endothelial cell.

In another embodiment, a further bioactive component is a collecting duct cell. In yet another embodiment, a further bioactive component is a smooth muscle cell. In another embodiment, a further bioactive component is a mesenchymal stem cell. In another embodiment, a further bioactive component is a progenitor of endothelial, mesenchymal, epithelial or hematopoietic lineage. In another embodiment, a further bioactive component is a progenitor of endodermal, ectodermal or mesenchymal embryonic origin. In certain embodiments, a further bioactive component is a stem cell of any origin or derivation, including but not limited to embryonic (ES) and induced pluripotent (IPS) stem cell. In some embodiments, a further bioactive component is a derivative of a stem cell of any origin or derivation, including but not limited to embryonic (ES) and induced pluripotent (iPS) stem cell, wherein the derivative may be generated by the directed differentiation of the stem cell by defined combinations or cocktails of small molecules and/or protein and/or nucleic acid molecules. In one embodiment, a further bioactive component is a derivative of a progenitor of endothelial, mesenchymal, epithelial or hematopoietic lineage wherein the derivative may generated by the directed differentiation of the stem cell by defined combinations or cocktails of small molecules and/or protein and/or nucleic acid molecules, In one embodiment, a further bioactive component is a derivative of a progenitor of endodermal, ectodermal or mesenchymal embryonic origin wherein the derivative may be generated by the directed differentiation of the stem cell by defined combinations or cocktails of small molecules and/or protein and/or nucleic acid molecules. In one embodiment, a further bioactive component is a genetically modified cell of any lineage or derivation.

In still another embodiment, a further bioactive component is an intersitial cell. In one embodiment, the interstitial cell is a supportive fibroblast. In another embodiment, the interstitial cell is a specialized cortical erythropoietin-producing fibroblast.

In one embodiment, the further bioactive component is derived from a source that is autologous to the subject. In one other embodiment, the further bioactive component is derived from a source that is allogeneic to the subject. In certain embodiments, a further bioactive component is derived from a source that is autologous to the subject, which further bioactive component is a combined admixture with a still further bioactive components which are derived from a source that is allogeneic to the subject.

The invention provides, in certain aspects, methods for the targeted regeneration of renal mass and functionality by directed delivery of the cell populations and/or organoids and/or biomaterials described herein. The invention further provides, in other aspects, methods method for the rescue and/or recovery of renal functionality in patients having acute or chronic renal disease by administration of cell populations and/or organoids and/or biomaterials described herein.

### Therapeutic Organoids

The instant invention further provides organoids comprising and/or formed from the bioactive components described herein, *e.g*., B2, 84, and B3, which are depleted of inactive or undesired components, *e.g.,* B1 and B5, alone or admixed for use in the treatment of acute and/or chronic kidney disease. In one aspect, the present invention provides organiods comprising and/or formed from a specific subfraction, 84, depleted of or deficient in one or more cell types, *e.g*., vascular, endocrine, or endothelial, *i.e*., B4', retains therapeutic properties, *e.g.,* stabilization and/or improvement and/or regeneration of kidney function, alone or when admixed with other bioactive subtractions, *e.g.,* B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4. In all embodiments, the organoids of the invention are formed and cultured *ex vivo.* In all embodiments, the organoids may further comprise and/or be formed from further bioactive cell populations, including but not limited to, endothelial cells, endothelial progenitors, mesenchymal stem cells, adipose-derived progenitors, In one embodiment, the further bioactive cell populations, including but not limted to, endothelial cells, endothelial progenitors, mesenchymal stem cells, adipose-derived progenitors are admixed with the isolated, heterogeneous populations of kidney cells, enriched for specific bioactive components or cell types and/or depleted of specific inactive or undesired components or cell types.

In one embodiment, the organoids of the invention comprise or are formed from a B2 cell population, wherein the B2 cell population comprises an enriched population of tubular cells. In another embodiment, the heterogenous renal cell population further comprises a B4 cell population. In yet another embodiment, the heterogeneous renal cell population further comprises a B3 population. In still another embodiment, the heterogeneous renal cell population further comprises a B5 population.

In certain embodiments, the cell population comprises a B2 cell population, wherein the B2 cell population comprises an enriched population of tubular cells, and is depleted of a B1 cell population, and/or a B5 cell population.

In another aspect, the invention provides methods of forming organoids comprising and/or formed from bioactive components of the invention, *e.g*., B2, B4, and B3, which are depleted of inactive or undesired components, *e.g*., B1 and B5, alone or admixed. n one aspect, the present invention provides organiods comprised and/or formed from a specific subtraction, B4, depleted of or deficient in one or more cell types, *e.g.,* vascular, endocrine, or endothelial, *i.e*., B4', retains therapeutic properties, *e.g.,* stabilization and/or improvement and/or regeneration of kidney function, alone or when admixed with other bioactive subfractions, *e.g*., B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4.

In one embodiment, the organoids of the invention comprise and/or are formed from a B2 cell population, wherein the B2 cell population comprises an enriched population of tubular cells. In another embodiment, the heterogenous renal cell population further comprises a B4 cell population. In yet another embodiment, the heterogeneous renal cell population further comprises a B3 population. In still another embodiment, the heterogeneous renal cell population further comprises a B5 population.

in certain embodiments, the cell population comprises a B2 cell population, wherein the B2 cell population comprises an enriched population of tubular cells, and is depleted of a B1 cell population, and/or a B5 cell population.

In another aspect, the present invention provides methods of forming organoids using the bioactive cell preparations and/or admixtures described herein. General methods for generating tubules from primary renal cell populations using 3D COL(I) gel culture are known in the art, for example, as in Joraku et al., Methods, 2009 Feb;47(2):129-33. In all embodiments, the organoids of the invention are formed and cultured *ex vivo,*

In some embodiments, formation of organoids and tubules from the bioactive cell preparations and/or admixtures described herein may be induced, for example and without limitation, using the following culture methods or systems: i) 2D culture; ii) 3D culture: COL(I) gel; iii) 3D culture: Matrigel; iv) 3D culture: spinners, then COL(I)/Matrigel; and v) 3D culture: COL(IV) gel. Specific examples of formation of organoids and tubules from NKA are provided in 2 and 4 below.

In one embodiment, organoids formed from the bioactive cell preparations and/or admixtures described herein may be induced in 2D culture. In one embodiment, the bioactive cell preparations and/or admixtures described herein are seeded on standard 2D plastic-ware. In one embodiment, cells are seeded at a density of about 5000 cells/cm². Cells may be seeded in an appropriate medium, such as, for example Renal Cell Complete Growth Media (RCGM). In general, cell populations may be grown past confluence for about 7, 8, 9, 10, 11, 12, 13, 14, 15 days or more, with regular changes of media about every 3-4 days. In one embodiment, cells demonstrate spontaneous self-organization into spheroidal structures, *i.e*., organoids, and tubules between about 7 to about 15 days.

In another embodinment, organoids formed from the bioactive cell preparations and/or admixtures described herein may be induced in 3D culture. In one embodiment, the organoid is generated with the cell populations of the invention together with a biomaterial scaffold of natural or synthetic origin. In one embodiment, formulated the bioactive cell preparations and/or admixtures described herein may be incorporated into a collagen (I) gel, collagen (IV) gel, Matrigel or a mixture of any of these as previously described (see Guimaraes-Souza et al., 2012. In vitro reconstitution of human kidney structures for renal cell therapy. Nephrol Dial Transplant 0: 1-9). The liquid gel may be brought to a neutral pH and the bioactive cell preparations and/or admixtures described herein mixed in at about 500-2500 cells/ul. In one embodiment, about 1000 cells/ul are mixed in. The cell/gel mixture may be aliquoted into a well of a 24 well plate, for example, (about 200 to about 400ul/well) and allowed to solidify at 37 degrees C for several hours. Cell culture media may then added and the cultures allowed to mature for about 4, about 5, about 6, about 7, about 8, about 9, or about 10 days with regular changes of media. In one embodiment networks of tubular structures organize as lattices and rings form throughout the gel matrix by the bioactive cell preparations and/or admixtures described herein.

In another embodiment, organoids may be formed by suspension culture of the bioactive cell preparations and/or admixtures described herein in spinner flasks or low-bind plasticware. In one embodiment, cells may be cultured in media in spinner flasks for up to 4 days at about 80rpm. Spheroids may then be further cultured for about 7, about 8, about 9, or about 10 days on Matrigel coated plates, for example. In one embodiment, spheroids formed from the bioactive cell preparations and/or admixtures described herein show tubulogenic potential as shown by de novo budding of tubular structures from cultured spheroids.

### Cell populations

The SRC+ cell populations and/or organoids of the present invention may contain and/or be formed from isolated, heterogeneous populations of kidney cells, and admixtures thereof, enriched for specific bioactive components or cell types and/or depleted of specific inactive or undesired components or cell types for use in the treatment of kidney disease, *i.e*., providing stabilization and/or improvement and/or regeneration of kidney function, were previously described in Presnell et al. U.S. 2011-0117162 and Ilagsn et al. PCT/US2011/036347, the entire contents of which are incorporated herein by reference. The organoids may contain isolated renal cell fractions that lack cellular components as compared to a healthy individual yet retain therapeutic properties, *i.e*., provide stabilization and/or improvement and/or regeneration of kidney function. The cell populations, cell fractions, and/or admixtures of cells described herein may be derived from healthy individuals, individuals with a kidney disease, or subjects as described herein.

### Bioactive cell populations

The present invention contemplates SRC+ cell populations and therapeutic organoids comprising bioactive cell populations that are to be administered to target organs or tissue in a subject in need. A bioactive cell population generally refers to a cell population potentially having therapeutic properties upon administration to a subject. For example, upon administration to a subject in need, a organoid comprising a bioactive renal cell population can provide stabilization and/or improvement and/or regeneration of kidney function in the subject. The therapeutic properties may include a regenerative effect.

Bioactive cell populations include, without limitation, stem cells (*e.g.,* pluripotent, multipotent, oligopotent, or unipotent) such as embryonic stem cells, amniotic stem cells, adult stem cells (*e.g.,* hematopoietic, mammary, intestinal, mesenchymal, placental, lung, bone marrow, blood, umbilical cord, endothelial, dental pulp, adipose, neural, olfactory, neural crest, testicular), induced pluripotent stem cells; genetically modified cells; as well as cell populations or tissue explants derived from any source of the body. The bioactive cell populations may be isolated, enriched, purified, homogeneous, or heterogeneous in nature. Those of ordinary skill in the art will appreciate other bioactive cell populations that are suitable for use in generating the organoids of the present invention.

In one embodiment, the source of cells is the same as the intended target organ or tissue. For example, renal cells may be sourced from the kidney to generate an organoid to be administered to the kidney. In another embodiment, the source of cells is not the same as the intended target organ or tissue. For example, erythropoietin-expressing cells may be sourced from renal adipose to generate an organoid to be administered to the kidney.

In one aspect, the present invention provides organoids comprising certain subtractions of a heterogeneous population of renal cells, enriched for bioactive components and depleted of inactive or undesired components provide superior therapeutic and regenerative outcomes than the starting population. For example, bioactive renal cells described herein, *e.g.,* B2, B4, and B3, which are depleted of inactive or undesired components, *e.g*., B1 and B5, alone or admixed, can be used to generate an organoid to be used for the stabilization and/or improvement and/or regeneration of kidney function.

In another aspect, the organoids contain a specific subfraction, 84, depleted of or deficient in one or more cell types, *e.g.,* vascular, endocrine, or endothelial, *i.e*., B4', that retain therapeutic properties, *e.g.,* stabilization and/or improvement and/or regeneration of kidney function, alone or when admixed with other bioactive subtractions, *e.g.,* B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4.

The B2 cell population is characterized by expression of a tubular ceil marker selected from the group consisting of one or more of the following: megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (Slc9a4), aldehyde dehydrogenase 3 family, member B1 (Aldh3b1), aldehyde dehydrogenase 1 family, member A3 (Aldh1a3), and Calpain-8 (Capn8), and collecting duct marker Aquapcsrin-4 (Aqp4). B2 is larger and more granulated than B3 and/or B4 and thus having a buoyant density between about 1.045 g/ml and about 1.063 g/ml (rodent), between about 1.045 g/ml and 1.052 g/ml (human), and between about 1.045 g/ml and about 1.058 g/ml (canine).

The B3 cell population is characterized by the expression of vascular, glomerular and proximal tubular markers with some EPO-producing cells, being of an intermediate size and granularity in comparison to B2 and B4, and thus having a buoyant density between about 1.063 g/ml and about 1.073 g/ml (rodent), between about 1.052 g/ml and about 1.063 g/ml (human), and between about 1.058 g/ml and about 1.063 g/ml (canine), B3 is characterized by expression of markers selected from the group consisting of one or more of the following: aquaporin 7 (Aqp7), FXYD domain-containing ion transport regulator 2 (Fxyd2), solute carrier family 17 (sodium phosphate), member 3 (SIc17a3), solute carrier family 3, member 1 (Slc3a1), claudin 2 (Cldn2), napsin A aspartic peptidase (Napsa), solute carrier family 2 (facilitated glucose transporter), member 2 (Slc2a2), alanyl (membrane) aminopeptidase (Anpep), transmembrane protein 27 (Tmem27), acyl-CoA synthetase medium-chain family member 2 (Acsm2), glutathione peroxidase 3 (Gpx3), fructose-1,6- biphosphatase 1 (Fbp1), and alanine-glyoxylate aminotransferase 2 (Agxt2). B3 is also characterized by the vascular expression marker Platelet endothelial cell adhesion molecule (Pecam) and the glomerular expression marker podocin (Podn).

The B4 cell population is characterized by the expression of a vascular marker set containing one or more of the following: PECAM, VEGF, KDR, HIF1a, CD31, CD146,, a glomerular marker set containing one or more of the following: Podocin (Podn), and Nephrin (Neph); and an oxygen-tunable EPO enriched population compared to unfractionated (UNFX), B2 and B3, B4 is also characterized by the expression of one or more of the following markers: chemokine (C-X-C motif) receptor 4 (Cxcr4), endothelin receptor type B (Ednrb), collagen, type V, alpha 2 (Col5a2), Cadherin 5 (Cdh5), plasminogen activator, tissue (Plat), angiopoietin 2 (Angpt2), kinase insert domain protein receptor (Kdr), secreted protein, acidic, cysteine-rich (osteonectin) (Sparc), serglycin (Srgn), T!MP metallopeptidase inhibitor 3 (Timp3), Wilms tumor 1 (Wt1), wingless-type MMTV integration site family, member 4 (Wnt4), regulator of G-protein signaling 4 (Rgs4), Platelet endothelial cell adhesion molecule (Pecam), and Erythropoietin (Epo). 84 is also characterized by smaller, less granulated cells compared to either B2 or B3, with a buoyant density between about 1.073 g/ml and about 1.091g/ml (rodent), between about 1.063 g/ml and about 1.091 g/mL (human and canine).

The B4' cell population is defined as having a buoyant density of between 1.063 g/ml and 1.091 g/ml and expressing one or more of the following markers: PECAM, vEGF, KDR, HIF1a, podocin, nephrin, EPO, CK7, CK8/18/19. in one embodiment, the B4' cell population is characterized by the expression of a vascular marker set containing one or more of the following: PECAM, vEGF, KDR, HIF1a, CD31, CD146. In another embodiment, the B4' cell population is characterized by the expression of an endocrine marker EPO. In one embodiment, the B4' cell population is characterized by the expression of a glomerular marker set containing one or more of the following: Podocin (Podn), and Nephrin (Neph). In certain embodiments, the B4' cell population is characterized by the expression of a vascular marker set containing one or more of the following: PECAM, vEGF, KDR, HIF1a and by the expression of an endocrine marker EPO. In another embodiment, B4' is also characterized by smaller, less granulated cells compared to either B2 or B3, with a buoyant density between about 1.073 g/ml and about 1.091g/ml (rodent), between about 1.063 g/ml and about 1.091 g/ml (human and canine).

In one aspect, the present invention provides organoids containing an isolated, enriched B4' population of human renal cells comprising at least one of erythropoietin (EPO)-producing cells, vascular cells, and glomerular cells having a density between 1.063 g/ml and 1.091 g/mL. In one embodiment, the B4' cell population is characterized by expression of a vascular marker. In certain embodiments, the B4' cell population is not characterized by expression of a glomerular marker. In some embodiments, the B4' cell population is capable of oxygen-tunable erythropoietin (EPO) expression.

In one embodiment, the organoid of the invention contains the B4' cell population but does not include a B2 cell population comprising tubular cells having a density between 1.045 g/mL and 1.052 g/mL. In another embodiment, the B4' cell population-containing organoid does not include a B1 cell population comprising large granular cells of the collecting duct and tubular system having a density of < 1.045 g/m!. In yet another embodiment, the B4' cell population organoid does not include a B5 cell population comprising debris and small cells of low granularity and viability with a density > 1.091 g/ml.

In one embodiment, the B4' cell population-containing organoid does not include a B2 cell population comprising tubular cells having a density between 1.045 g/ml and 1.052 g/ml; a 81 cell population comprising large granular cells of the collecting duct and tubular system having a density of < 1.045 g/ml; and a B5 cell population comprising debris and small cells of low granularity and viability with a density > 1.091 g/ml. In some embodiments, the B4' cell population may be derived from a subject having kidney disease.

In one aspect, the present invention provides organoids containing admixtures of human renal cells comprising a first cell population, B2, comprising an isolated, enriched population of tubular cells having a density between 1.045 g/ml and 1.052 g/ml, and a second cell population, B4', comprising erythropoietin (EPO)-producing cells and vascular cells but depleted of glomerular cells having a density between about 1.063 g/ml and 1.091 g/mL, wherein the admixture does not include a B1 cell population comprising large granular cells of the collecting duct and tubular system having a density of < 1.045 g/ml, or a B5 cell population comprising debris and small cells of low granularity and viability with a density > 1.091 g/m!. In certain embodiment, the B4' cell population is characterized by expression of a vascular marker. In one embodiment, the B4' cell population is not characterized by expression of a glomerular marker. In certain embodiments, B2 further comprises collecting duct epithelial cells. In one embodiment, the organoid contains or is formed from an admixture of cells that is capable of receptor-mediated albumin uptake. In another embodiment, the admixture of cells is capable of oxygen-tunable erythropoietin (EPO) expression. In one embodiment, the admixture contains HAS-2-expressing cells capable of producing and/or stimulating the production of high-molecular weight species of hyaluronic acid (HA) both *in vitro* and *in vivo,* In all embodiments, the first and second cell populations may be derived from kidney tissue or cultured kidney cells (Basu et al, Lipids in Health and Disease, 2011, 10:171).

In one embodiment, the organoid contains an admixture that is capable of providing a regenerative stimulus upon *in vivo* delivery, In other embodiments, the admixture is capable of reducing the decline of, stabilizing, or improving glomerular filtration, tubular resorption, urine production, and/or endocrine function upon *in vivo* delivery. In one embodiment, the B4' cell population is derived from a subject having kidney disease.

In one aspect, the present invention provides organoids containing an isolated, enriched B4' population of human renal cells comprising at least one of erythropoietin (EPO)-producing cells, vascular cells, and glomerular cells having a density between 1.063 g/ml and 1.091 g/mL. In one embodiment, the B4' cell population is characterized by expression of a vascular marker. In certain embodiments, the B4' cell population is not characterized by expression of a glomerular marker. The glomerular marker that is not expressed may be podocin (see Example 10). In some embodiments, the B4' cell population is capable of oxygen-tunable erythropoietin (EPO) expression.

In one embodiment, the B4' cell population-containing organoid does not include a B2 cell population comprising tubular cells having a density between 1.045 g/ml and 1.052 g/mL. In another embodiment, the B4' cell population organoid does not include a B1 cell population comprising large granular cells of the collecting duct and tubular system having a density of < 1.045 g/ml. In yet another embodiment, the B4' cell population organoid does not include a B5 cell population comprising debris and small cells of low granularity and viability with a density > 1.091 g/ml.

In one embodiment, the B4' cell population-containing organoid does not include a B2 cell population comprising tubular cells having a density between 1.045 g/ml and 1.052 g/ml; a B1 cell population comprising large granular cells of the collecting duct and tubular system having a density of < 1.045 g/ml; and a B5 cell population comprising debris and small cells of low granularity and viability with a density > 1.091 g/ml. In some embodiments, the B4' cell population may be derived from a subject having kidney disease.

In one aspect, the present invention provides organoids containing an admixture of human renal cells comprising a first cell population, B2, comprising an isolated, enriched population of tubular cells having a density between 1.045 g/ml and 1.052 g/mL, and a second cell population, B4', comprising erythropoietin (EPO)-producing cells and vascular cells but depleted of glomerular cells having a density between about 1.063 g/ml and 1.091 g/ml, wherein the admixture does not include a B1 cell population comprising large granular cells of the collecting duct and tubular system having a density of < 1.045 g/ml, or a B5 cell population comprising debris and small cells of low granularity and viability with a density > 1.091 g/ml. In certain embodiment, the B4' cell population is characterized by expression of a vascular marker. In one embodiment, the B4' cell population is not characterized by expression of a glomerular marker. In certain embodiments, B2 further comprises collecting duct epithelial cells, In one embodiment, the admixture of cells is capable of receptor-mediated albumin uptake. In another embodiment, the admixture of cells is capable of oxygen-tunable erythropoietin (EPO) expression. In one embodiment, the admixture contains HAS-2-expressing cells capable of producing and/or stimulating the production of high-molecular weight species of hyaluronic acid (HA) both *in vitro* and *in vivo.* In all embodiments, the first and second cell populations may be derived from kidney tissue or cultured kidney cells.

In another aspect, the present invention provides organiods containing a heterogeneous renal cell population comprising a combination of cell fractions or enriched cell populations (e.g., B1, B2, B3, B4 (or B4'), and B5). In one embodiment, the combination has a buoyant density between about 1.045 g/ml and about 1.091 g/ml. In one other embodiment, the combination has a buoyant density between less than about 1.045 g/ml and about 1.099 g/ml or about 1.100 g/ml. In another embodiment, the combination has a buoyant density as determined by separation on a density gradient, *e.g.,* by centrifugation. In yet another embodiment, the combination of cell fractions contains B2, B3, and B4 (or 84') depleted of B1 and/or B5. In some embodiments, the combination of cell fractions contains 82, B3, B4 (or B4'), and B5 but is depleted of B1. Once depleted of B1 and/or B5, the combination may be subsequently cultured *in vitro* prior to the preparation of an organoid comprising the combination of B2, B3, and B4 (or B4') cell fractions.

The inventors of the present invention have surprisingly discovered that *in vitro* culturing of a B1-depleted combination of B2, B3, B4, and B5 results in depletion of B5. In one embodiment, B5 is depleted after at least one, two, three, four, or five passages. In one other embodiment, the B2, B3, B4, and B5 cell fraction combination that is passaged under the conditions described herein provides a passaged cell population having B5 at a percentage that is less than about 5%, less than about 4%, less than about 3%, less than about 2%, less than about 1%, or less than about 0.5% of the passaged cell population.

In another embodiment, B4' is part of the combination of cell fractions. In one other embodiment, the *in vitro* culturing depletion of 85 is under hypoxic conditions.

In one embodiment, the organoid contains an admixture that is capable of providing a regenerative stimulus upon *in vivo* delivery. In other embodiments, the admixture is capable of reducing the decline of, stabilizing, or improving glomerular filtration, tubular resorption, urine production, and/or endocrine function upon *in vivo* delivery. In one embodiment, the B4' cell population is derived from a subject having kidney disease.

In a preferred embodiment, the organoid contains and/or is formed from an admixture that comprises B2 in combination with B3 and/or B4. In another preferred embodiment, the admixture comprises B2 in combination with B3 and/or B4'. In other preferred embodiments, the admixture consists of or consists essentially of (i) B2 in combination with B3 and/or B4; or (II) B2 in combination with B3 and/or B4'.

The admixtures that contain a B4' cell population may contain B2 and/or B3 cell populations that are also obtained from a non-healthy subject. The non-healthy subject may be the same subject from which the B4' fraction was obtained. In contrast to the B4' cell population, the B2 and B3 cell populations obtained from non-healthy subjects are typically not deficient in one or more specific cell types as compared to a starting kidney cell population derived from a healthy individual.

As described in Presnell et al. WO/2010/056328, it has been found that the B2 and B4 cell preparations are capable of expressing higher molecular weight species of hyaluronic acid (HA) both *in vitro* and *in vivo,* through the actions of hyaluronic acid synthase-2 (HAS-2) - a marker that is enriched more specifically in the B2 cell population. Treatment with B2 in a 5/6 Nx model was shown to reduce fibrosis, concomitant with strong expression HAS-2 expression *in vivo* and the expected production of high-molecular-weight HA within the treated tissue. Notably, the 5/6 Na model left untreated resulted in fibrosis with limited detection of HAS-2 and little production of high-molecular-weight HA. Without wishing to be bound by theory, it is hypothesized that this anti-inflammatory high-molecular weight species of HA produced predominantly by B2 (and to some degree by B4) acts synergistically with the cell preparations in the reduction of renal fibrosis and in the aid of renal regeneration. Accordingly, the instant invention includes organoids containing the bioactive renal cells described herein along with a biomaterial comprising hyaluronic acid. Also contemplated by the instant invention is the provision of a biomaterial component of the regenerative stimulus via direct production or stimulation of production by the implanted cells.

In one aspect, the present invention provides organoids containing and/or generated from isolated, heterogeneous populations of EPO-producing kidney cells for use in the treatment of kidney disease, anemia and/or EPO deficiency in a subject in need. In one embodiment, the cell populations are derived from a kidney biopsy. In another embodiment, the cell populations are derived from whole kidney tissue. In one other embodiment, the cell populations are derived from in vitro cultures of mammalian kidney cells, established from kidney biopsies or whole kidney tissue. In all embodiments, these populations are unfractionated cell populations, also referred to herein as non-enriched cell populations.

In another aspect, the present invention provides organoids containing and/or generated from isolated populations of erythropoietin (EPO)-producing kidney cells that are further enriched such that the proportion of EPO-producing cells in the enriched subpopulation is greater relative to the proportion of EPO-producing cells in the starting or initial cell population. In one embodiment, the enriched EPO-producing cell fraction contains a greater proportion of interstitial fibroblasts and a lesser proportion of tubular cells relative to the interstitial fibroblasts and tubular cells contained in the unenriched initial population. In certain embodiments, the enriched EPO-producing cell fraction contains a greater proportion of glomerular cells and vascular cells and a lesser proportion of collecting duct cells relative to the glomerular cells, vascular cells and collecting duct cells contained in the unenriched initial population, In such embodiments, these populations are referred to herein as the "B4" cell population.

In another aspect, the present invention provides organoids containing and/or generated from an EPO-producing kidney cell population that is admixed with one or more additional kidney cell populations. In one embodiment, the EPO-producing cell population is a first cell population enriched for EPO-producing cells, e.g., B4. In another embodiment, the EPO-producing cell population is a first cell population that is not enriched for EPO-producing cells, e.g., B2. In another embodiment, the first cell population is admixed with a second kidney cell population. In some embodiments, the second cell population is enriched for tubular cells, which may be demonstrated by the presence of a tubular cell phenotype. In another embodiment, the tubular cell phenotype may be indicated by the presence of one tubular cell marker. In another embodiment, the tubular cell phenotype may be indicated by the presence of one or more tubular cell markers. The tubular cell markers include, without limitation, megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (Slc9a4), aldehyde dehydrogenase 3 family, member B1 (Aldh3b1), aldehyde dehydrogenase 1 family, member A3 (Aldhla3), and Calpain-8 (CapnS). In another embodiment, the first cell population is admixed with at least one of several types of kidney cells including, without limitation, interstitium-derived cells, tubular cells, collecting duct-derived cells, glomerulus-derived cells, and/or cells derived from the blood or vasculature.

The organoids of the present invention may include or be formed from EPO-producing kidney cell populations containing B4 or B4' in the form of an admixture with 82 and/or B3, or in the form of an enriched cell population, e.g., B2+B3+B4/B4'.

In one aspect, the organoids contain and/or are generated from EPO-producing kidney cell populations that are characterized by EPO expression and bioresponsiveness to oxygen, such that a reduction in the oxygen tension of the culture system results in an induction in the expression of EPO. In one embodiment, the EPO-producing cell populations are enriched for EPO-producing cells. In one embodiment, the EPO expression is induced when the cell population is cultured under conditions where the cells are subjected to a reduction in available oxygen levels in the culture system as compared to a cell population cultured at normal atmospheric (^{~}21%) levels of available oxygen. In one embodiment, EPO-producing cells cultured in lower oxygen conditions express greater levels of EPO relative to EPO-producing cells cultured at normal oxygen conditions. In general, the culturing of cells at reduced levels of available oxygen (also referred to as hypoxic culture conditions) means that the level of reduced oxygen is reduced relative to the culturing of cells at normal atmospheric levels of available oxygen (also referred to as normal or normoxic culture conditions). In one embodiment, hypoxic cell culture conditions include culturing cells at about less than 1% oxygen, about less than 2% oxygen, about less than 3% oxygen, about less than 4% oxygen, or about less than 5% oxygen. In another embodiment, normal or normoxic culture conditions include culturing cells at about 10% oxygen, about 12% oxygen, about 13% oxygen, about 14% oxygen, about 15% oxygen, about 16% oxygen, about 17% oxygen, about 18% oxygen, about 19% oxygen, about 20% oxygen, or about 21% oxygen,

In one other embodiment, induction or increased expression of EPO is obtained and can be observed by culturing cells at about less than 5% available oxygen and comparing EPO expression levels to cells cultured at atmospheric (about 21%) oxygen. In another embodiment, the induction of EPO is obtained in a culture of cells capable of expressing EPO by a method that includes a first culture phase in which the culture of cells is cultivated at atmospheric oxygen (about 21%) for some period of time and a second culture phase in which the available oxygen levels are reduced and the same cells are cultured at about less than 5% available oxygen. In another embodiment, the EPO expression that is responsive to hypoxic conditions is regulated by HlF1α. Those of ordinary skill in the art will appreciate that other oxygen manipulation culture conditions known in the art may be used for the cells described herein.

In one aspect, the organoid contains and/or is formed from enriched populations of EPO-producing mammalian cells characterized by bio-responsiveness (e.g., EPO expression) to perfusion conditions. In one embodiment, the perfusion conditions include transient, intermittent, or continuous fluid flow (perfusion). In one embodiment, the EPO expression is mechanically-induced when the media in which the cells are cultured is intermittently or continuously circulated or agitated in such a manner that dynamic forces are transferred to the cells via the flow. In one embodiment, the cells subjected to the transient, intermittent, or continuous fluid flow are cultured in such a manner that they are present as three-dimensional structures in or on a material that provides framework and/or space for such three-dimensional structures to form. In one embodiment, the cells are cultured on porous beads and subjected to intermittent or continuous fluid flow by means of a rocking platform, orbiting platform, or spinner flask. In another embodiment, the cells are cultured on three-dimensional scaffolding and placed into a device whereby the scaffold is stationary and fluid flows directionally through or across the scaffolding. Those of ordinary skill in the art will appreciate that other perfusion culture conditions known in the art may be used for the cells described herein.

### Inactive cell populations

As described herein, the present invention is based, in part, on the surprising finding that organoids comprising and/or formed from certain subtractions of a heterogeneous population of renal cells, enriched for bioactive components and depleted of inactive or undesired components, provide superior therapeutic and regenerative outcomes than the starting population. In preferred embodiments, the organoids provided by the present invention contain cellular populations that are depleted of B1 and/or B5 cell populations. For instance, the following may be depleted of B1 and/or B5: admixtures of two or more of B2, B3, and B4 (or B4'); an enriched cell population of B2, B3, and B4 (or B4').

The B1 cell population comprises large, granular cells of the collecting duct and tubular system, with the cells of the population having a buoyant density less than about 1.045 g/m. The B5 cell population is comprised of debris and small cells of low granularity and viability and having a buoyant density greater than about 1.091 g/ml.

### Methods of isolating and culturing cell populations

In one aspect, the SRC+ cell populations and/or organoids of the present invention contain and/or are formed from cell populations that have been isolated and/or cultured from kidney tissue. Methods are provided herein for separating and isolating the renal cellular components, e.g., enriched cell populations that are contained in the organoids for therapeutic use, including the treatment of kidney disease, anemia, EPO deficiency, tubular transport deficiency, and glomerular filtration deficiency. In one embodiment, the cell populations are isolated from freshly digested, i.e., mechanically or enzymatically digested, kidney tissue or from heterogeneous in vitro cultures of mammalian kidney cells. Methods for isolating the further bioactive cell populations which comprise the SRC+ cell populations of the invention are further described in the Examples.

The organoids may contain and/or are formed from heterogeneous mixtures of renal cells that have been cultured in hypoxic culture conditions prior to separation on a density gradient provides for enhanced distribution and composition of cells in both 84, including B4', and B2 and/or B3 fractions. The enrichment of oxygen-dependent cells in B4 from B2 was observed for renal cells isolated from both diseased and non-diseased kidneys. Without wishing to be bound by theory, this may be due to one or more of the following phenomena: 1) selective survival, death, or proliferation of specific cellular components during the hypoxic culture period; 2) alterations in cell granularity and/or size in response to the hypoxic culture, thereby effecting alterations in buoyant density and subsequent localization during density gradient separation; and 3) alterations in cell gene / protein expression in response to the hypoxic culture period, thereby resulting in differential characteristics of the cells within any given fraction of the gradient. Thus, in one embodiment, the organoids contain and/or are formed from cell populations enriched for tubular cells, e.g., B2, are hypoxia-resistant.

Exemplary techniques for separating and isolating the cell populations of the invention include separation on a density gradient based on the differential specific gravity of different cell types contained within the population of interest. The specific gravity of any given cell type can be influenced by the degree of granularity within the cells, the intracellular volume of water, and other factors. In one aspect, the present invention provides optimal gradient conditions for isolation of the cell preparations of the instant invention, *e.g.,* B2 and B4, including 84', across multiple species including, but not limited to, human, canine., and rodent. In a preferred embodiment, a density gradient is used to obtain a novel enriched population of tubular cells fraction, *i*.*e*., B2 cell population, derived from a heterogeneous population of renal cells, In one embodiment, a density gradient is used to obtain a novel enriched population of EPO-producing cells fraction, i.e., B4 cell population, derived from a heterogeneous population of renal cells, In other embodiments, a density gradient is used to obtain enriched subpopulations of tubular cells, glomerular cells, and endothelial cells of the kidney. In one embodiment, both the EPO-producing and the tubular cells are separated from the red blood cells and cellular debris. In one embodiment, the EPO-producing, glomerular, and vascular cells are separated from other cell types and from red blood cells and cellular debris, while a subpopulation of tubular cells and collecting duct cells are concomitantly separated from other cell types and from red blood cells and cellular debris. In one other embodiment, the endocrine, glomerular, and/or vascular cells are separated from other cell types and from red blood cells and cellular debris, while a subpopulation of tubular cells and collecting duct cells are concomitantly separated from other cell types and from red blood cells and cellular debris.

In one aspect, the the organoids of the present invention contain and/or are formed from cell populations generated by using, in part, the OPTIPREP^{®} (Axis-Shield) density gradient medium, comprising 60% nonionic iodinated compound iodixanol in water, based on certain key features described below. One of skill in the art, however, will recognize that any density gradient or other means, *e.g.,* immunological separation using cell surface markers known in the art, comprising necessary features for isolating the cell populations of the instant invention may be used in accordance with the invention. It should also be recognized by one skilled in the art that the same cellular features that contribute to separation of cellular subpopulations via density gradients (size and granularity) can be exploited to separate cellular subpopulations via flow cytometry (forward scatter = a reflection of size via flow cytometry, and side scatter = a reflection of granularity). Importantly, the density gradient medium should have low toxicity towards the specific cells of interest. While the density gradient medium should have low toxicity toward the specific cells of interest, the instant invention contemplates the use of gradient mediums which play a role in the selection process of the cells of interest. Without wishing to be bound by theory, it appears that the cell populations of the instant invention recovered by the gradient comprising iodixanol are iodixanol-resistant, as there is an appreciable loss of cells between the loading and recovery steps, suggesting that exposure to iodixanol under the conditions of the gradient leads to elimination of certain cells. The cells appearing in the specific bands after the iodixanol gradient are resistant to any untoward effects of iodixanol and/or density gradient exposure. Accordingly, the use of additional contrast media which are mild to moderate nephrotoxins in the isolation and/or selection of the cell populations for the organoids described herein is also contemplated. In addition, the density gradient medium should also not bind to proteins in human plasma or adversely affect key functions of the cells of interest.

in another aspect, the present invention provides organoids containing and/or formed from cell populations that have been enriched and/or depleted of kidney cell types using fluorescent activated cell sorting (FACS). In one embodiment, kidney cell types may be enriched and/or depleted using BD FACSAria^{™} or equivalent.

In another aspect, the organoids contain and/or are formed from cell populations that have been enriched and/or depleted of kidney cell types using magnetic cell sorting. In one embodiment, kidney cell types may be enriched and/or depleted using the Miltenyi autoMACS^{®} system or equivalent.

In another aspect, the organoids may include and/or may be formed from renal cell populations that have been subject to three-dimensional culturing. In one aspect, the methods of culturing the cell populations are via continuous perfusion. In one embodiment, the cell populations cultured via three-dimensional culturing and continuous perfusion demonstrate greater cellularity and interconnectivity when compared to cell populations cultured statically. In another embodiment, the cell populations cultured via three dimensional culturing and continuous perfusion demonstrate greater expression of EPO, as well as enhanced expression of renal tubule-associate genes such as e-cadherin when compared to static cultures of such cell populations. In yet another embodiment, the cell populations cultured via continuous perfusion demonstrate greater levels of glucose and glutamine consumption when compared to cell populations cultured statically.

As described herein, low or hypoxic oxygen conditions may be used in the methods to prepare the cell populations for the organoids of the present invention. However, the methods of preparing cell populations may be used without the step of low oxygen conditioning. In one embodiment, normoxic conditions may be used.

Those of ordinary skill in the art will appreciate that other methods of isolation and culturing known in the art may be used for the cells described herein.

### Biomaterials

As described in Bertram et al. U.S. Published Application 20070276507, polymeric matrices or scaffolds may be shaped into any number of desirable configurations to satisfy any number of overall system, geometry or space restrictions. In one embodiment, the matrices or scaffolds of the present invention may be three-dimensional and shaped to conform to the dimensions and shapes of an organ or tissue structure. For example, in the use of the polymeric scaffold for treating kidney disease, anemia, EPO deficiency, tubular transport deficiency, or glomerular filtration deficiency, a three-dimensional (3-D) matrix may be used. A variety of differently shaped 3-D scaffolds may be used. Naturally, the polymeric matrix may be shaped in different sizes and shapes to conform to differently sized patients. The polymeric matrix may also be shaped in other ways to accommodate the special needs of the patient. In another embodiment, the polymeric matrix or scaffold may be a biocompatible, porous polymeric scaffold. The scaffolds may be formed from a variety of synthetic or naturally-occurring materials including, but not limited to, open-cell polylactic acid (OPLA^{®}), cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulfone, polyethylene, polyfiuoroolefin, polyimide, polyolefin, polyoxadiazole, polyphenylene oxide, polyphenylene sulfide, polypropylene, polystyrene, polysulfide, polysulfone, polytetrafluoroethylene, polythioether, polytriazole, polyurethane, polyvinyl, polyvinylidene fluoride, regenerated cellulose, silicone, urea-formaldehyde, collagens, laminins, fibronectin, glycosaminoglycans, silk, elastin, alginate, hyaluronic acid, agarose, or copolymers or physical blends thereof. Scaffolding configurations may range from liquid hydrogel suspensions to soft porous scaffolds to rigid, shape-holding porous scaffolds.

The scaffold may be composed of any material form of biomaterial including but not limited to diluents, cell carriers, micro-beads, material fragments, scaffolds of synthetic composition including but not limited to PGA, PLGA, PLLA, OPLA, electrospun nanofibers and foams of synthetic composition including but not limited to PGA, PLGA, PLLA, OPLA, electrospun nanofibers.

Hydrogels may be formed from a variety of polymeric materials and are useful in a variety of biomedical applications. Hydrogels can be described physically as three-dimensional networks of hydrophilic polymers, Depending on the type of hydrogel, they contain varying percentages of water, but altogether do not dissolve in water. Despite their high water content, hydrogels are capable of additionally binding great volumes of liquid due to the presence of hydrophilic residues, Hydrogels swell extensively without changing their gelatinous structure, The basic physical features of hydrogel can be specifically modified, according to the properties of the polymers used and the additional special equipments of the products.

Preferably, the hydrogel is made of a polymer, a biologically derived material, a synthetically derived material or combinations thereof, that is biologically inert and physiologically compatible with mammalian tissues. The hydrogel material preferably does not induce an inflammatory response. Examples of other materials which can be used to form a hydrogel include (a) modified alginates, (b) polysaccharides (e.g. gellan gum and carrageenans) which gel by exposure to monovalent cations, (c) polysaccharides (e.g., hyaluronic acid) that are very viscous liquids or are thixotropic and form a gel over time by the slow evolution of structure, and (d) polymeric hydrogel precursors (e.g., polyethylene oxide-polypropylene glycol block copolymers and proteins). U.S. Pat. No. 6,224,893 B1 provides a detailed description of the various polymers, and the chemical properties of such polymers, that are suitable for making hydrogels in accordance with the present invention.

Scaffolding or biomaterial characteristics may enable cells to attach and interact with the scaffolding or biomaterial material, and/or may provide porous spaces into which cells can be entrapped. In one embodiment, the porous scaffolds or biomaterials of the present invention allow for the addition or deposition of one or more populations or admixtures of cells on a biomaterial configured as a porous scaffold (e.g., by attachment of the cells) and/or within the pores of the scaffold (e.g., by entrapment of the cells). In another embodiment, the scaffolds or biomaterials allow or promote for cell:cell and/or cell:biomaterial interactions within the scaffold to form constructs as described herein.

in one embodiment, the biomaterial used in accordance with the present invention is comprised of hyaluronic acid (HA) in hydrogel form, containing HA molecules ranging in size from 5.1 kDA to >2 × 10⁶ kDa. In another embodiment, the biomaterial used in accordance with the present invention is comprised of hyaluronic acid in porous foam form, also containing HA molecules ranging in size from 5.1 kDA to >2 × 10⁶ kDa . In yet another embodiment, the biomaterial used in accordance with the present invention is comprised of of a poly-lactic acid (PLA)-based foam, having an open-cell structure and pore size of about 50 microns to about 300 microns. In yet another embodiment, the specific cell populations, preferentially B2 but also B4, provide directly and/or stimulate synthesis of high molecular weight Hyaluronic Acid through Hyaluronic Acid Synthase-2 (HAS-2), especially after intra-renal implantation.

The biomaterials described herein may also be designed or adapted to respond to certain external conditions, *e.g., in vitro* or *in vivo.* In one embodiment, the biomaterials are temperature-sensitive (*e.g.,* either *in vitro* or *in vivo*). In another embodiment, the biomaterials are adapted to respond to exposure to enzymatic degradation (*e*.*g*., either *in vitro* or *in vivo*). The biomaterials' response to external conditions can be fine tuned as described herein. Temperature sensitivity of the organoid described can be varied by adjusting the percentage of a biomaterial in the organoid. Alternatively, biomaterials may be chemically cross-linked to provide greater resistance to enzymatic degradation. For instance, a carbodiimide crosslinker may be used to chemically crosslink gelatin beads thereby providing a reduced susceptibility to endogenous enzymes.

In one aspect, the response by the biomaterial to external conditions concerns the loss of structural integrity of the biomaterial. Although temperature-sensitivity and resistance to enzymatic degradation are provided above, other mechanisms exist by which the loss of material integrity may occur in different biomaterials. These mechanisms may include, but are not limited to thermodynamic (e.g., a phase transition such as melting, diffusion (*e*.*g*., diffusion of an ionic crosslinker from a biomaterial into the surrounding tissue)), chemical, enzymatic, pH (e.g., pH-sensitive liposomes), ultrasound, and photolabile (light penetration). The exact mechanism by which the biomaterial loses structural integrity will vary but typically the mechanism is triggered either at the time of implantation or post-implantation.

Those of ordinary skill in the art will appreciate that other types of synthetic or naturally-occurring materials known in the art may be used to form scaffolds as described herein.

In one aspect, the present invention provides constructs as described herein made from the above-referenced scaffolds or biomaterials.

### Constructs

In one aspect, the invention provides organoids that contain implantable constructs having one or more of the cell populations described herein for the treatment of kidney disease, anemia, or EPO deficiency in a subject in need. In one embodiment, the construct is made up of a biocompatible material or biomaterial, scaffold or matrix composed of one or more synthetic or naturally-occurring biocompatible materials and one or more cell populations or admixtures of cells described herein deposited on or embedded in a surface of the scaffold by attachment and/or entrapment. In certain embodiments, the construct is made up of a biomaterial and one or more cell populations or admixtures of cells described herein coated with, deposited on, deposited in, attached to, entrapped in, embedded in, seeded, or combined with the biomaterial component(s). Any of the cell populations described herein, including enriched cell populations or admixtures thereof, may be used in combination with a matrix to form a construct.

In another embodiment, the deposited cell population or cellular component of the construct is a first kidney cell population enriched for oxygen-tunable EPO-producing cells, In another embodiment, the first kidney cell population contains glomerular and vascular cells in addition to the oxygen-tunable EPO-producing cells, In one embodiment, the first kidney cell population is a B4' cell population. In one other embodiment, the deposited cell population or cellular component(s) of the construct includes both the first enriched renal cell population and a second renal cell population. In some embodiments, the second cell population is not enriched for oxygen-tunable EPO producing cells. In another embodiment, the second cell population is enriched for renal tubular cells. In another embodiment, the second cell population is enriched for renal tubular cells and contains collecting duct epithelial cells. In other embodiments, the renal tubular cells are characterized by the expression of one or more tubular cell markers that may include, without limitation, megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (Slc9a4), aldehyde dehydrogenase 3 family, member B1 (Aldh3b1), aldehyde dehydrogenase 1 family, member A3 (Aldh1a3), and Calpain-8 (Capn8).

In one embodiment, the cell populations deposited on or combined with biomaterials or scaffolds to form constructs of the present invention are derived from a variety of sources, such as autologous sources. Non-autologous sources are also suitable for use, including without limitation, allogeneic, or syngeneic (autogeneic or isogeneic) sources.

Those of ordinary skill in the art will appreciate there are several suitable methods for depositing or otherwise combining cell populations with biomaterials to form a construct.

In one aspect, the constructs of the present invention are suitable for use in the methods of use described herein. In one embodiment, the constructs are suitable for administration to a subject in need of treatment for a kidney disease of any etiology, anemia, or EPO deficiency of any etiology. In other embodiments, the constructs are suitable for administration to a subject in need of an improvement in or restoration of erythroid homeostasis. In another embodiment, the constructs are suitable for administration to a subject in need of improved kidney function.

In yet another aspect, the present invention provides a construct for implantation into a subject in need of improved kidney function comprising: a) a biomaterial comprising one or more biocompatible synthetic polymers or naturally-occurring proteins or peptides; and b) an admixture of mammalian renal cells derived from a subject having kidney disease comprising a first cell population, B2, comprising an isolated, enriched population of tubular cells having a density between 1.045 g/ml and 1.052 g/mL and a second cell population, B4', comprising erythropoietin (EPO)-producing cells and vascular cells but depleted of glomerular cells having a density between 1.063 g/mL and 1.091 g/ml, coated with, deposited on or in, entrapped in, suspended in, embedded in and/or otherwise combined with the biomaterial. In certain embodiments, the admixture does not include a B1 cell population comprising large granular cells of the collecting duct and tubular system having a density of < 1.045 g/ml, or a B5 cell population comprising debris and small cells of low granularity and viability with a density > 1.091 g/ml.

In one embodiment, the construct includes a B4' cell population which is characterized by expression of a vascular marker. In some embodiments, the B4' cell population is not characterized by expression of a glomerular marker. In certain embodiments, the admixture is capable of oxygen-tunable erythropoietin (EPO) expression, In all embodiments, the admixture may be derived from mammalian kidney tissue or cultured kidney cells.

In one embodiment, the construct includes a biomaterial configured as a three-dimensional (3-D) porous biomaterial suitable for entrapment and/or attachment of the admixture. In another embodiment, the construct includes a biomaterial configured as a liquid or semi-liquid gel suitable for embedding, attaching, suspending, or coating mammalian cells, In yet another embodiment, the construct includes a biomaterial configured comprised of a predominantly high-molecular weight species of hyaluronic acid (HA) in hydrogel form. In another embodiment, the construct includes a biomaterial comprised of a predominantly high-molecular weight species of hyaluronic acid in porous foam form, In yet another embodiment, the construct includes a biomaterial comprised of a poly-lactic acid-based foam having pores of between about 50 microns to about 300 microns. In still another embodiment, the construct includes one or more cell populations that may be derived from a kidney sample that is autologous to the subject in need of improved kidney function. In certain embodiments, the sample is a kidney biopsy. In some embodiments, the subject has a kidney disease. In yet other embodiments, the cell population is derived from a non-autologous kidney sample. In one embodiment, the construct provides erythroid homeostasis.

### Methods of use

In one aspect, the present invention provides methods for the treatment of a kidney disease, anemia, or EPO deficiency in a subject in need with SRC+ cell populations and/or organoids containing and/or formed from the kidney cell populations and admixtures of kidney cells described herein. In one embodiment, the method comprises administering to the subject an organoid(s) that includes and/or is formed from a first kidney cell population enriched for EPO-producing cells. In another embodiment, the first cell population is enriched for EPO-producing cells, glomerular cells, and vascular cells. In another embodiment, the oraganoid(s) may further include and/or may be formed from one or more additional kidney cell populations. In one embodiment, the additional cell population is a second cell population not enriched for EPO-producing cells. In another embodiment, the additional cell population is a second cell population not enriched for EPO-producing cells, glomerular cells, or vascular cells. In another embodiment, the organiod(s) also includes and/or is formed from a kidney cell population or admixture of kidney cells deposited in, deposited on, embedded in, coated with, or entrapped in a biomaterial to form an implantable construct, as described herein, for the treatment of a disease or disorder described herein. In one embodiment, the organoids are used alone or in combination with other cells or biomaterials, e.g., hydrogels, porous scaffolds, or native or synthetic peptides or proteins, to stimulate regeneration in acute or chronic disease states.

In another aspect, the effective treatment of a kidney disease, anemia, or EPO deficiency in a subject by the methods of the present invention can be observed through various indicators of erythropoiesis and/or kidney function. In one embodiment, the indicators of erythroid homeostasis include, without limitation, hematocrit (HCT), hemoglobin (HB), mean corpuscular hemoglobin (MCH), red blood cell count (RBC), reticulocyte number, reticulocyte %, mean corpuscular volume (MCV), and red blood cell distribution width (ROW). In one other embodiment, the indicators of kidney function include, without limitation, serum albumin, albumin to globulin ratio (A/G ratio), serum phosphorous, serum sodium, kidney size (measurable by ultrasound), serum calcium, phosphorous:calcium ratio, serum potassium, proteinuria, urine creatinine, serum creatinine, blood nitrogen urea (BUN), cholesterol levels, triglyceride levels and glomerular filtration rate (GFR). Furthermore, several indicators of general health and well-being include, without limitation, weight gain or loss, survival, blood pressure (mean systemic blood pressure, diastolic blood pressure, or systolic blood pressure), and physical endurance performance.

In another embodiment, an effective treatment with SRC+ cell populations or bioactive renal cell organoids is evidenced by stabilization of one or more indicators of kidney function. The stabilization of kidney function is demonstrated by the observation of a change in an indicator in a subject treated by a method of the present invention as compared to the same indicator in a subject that has not been treated by a method of the present invention. Alternatively, the stabilization of kidney function may be demonstrated by the observation of a change in an indicator in a subject treated by a method of the present invention as compared to the same indicator in the same subject prior to treatment. The change in the first indicator may be an increase or a decrease in value. In one embodiment, the treatment provided by the present invention may include stabilization of blood urea nitrogen (BUN) levels in a subject where the BUN levels observed in the subject are lower as compared to a subject with a similar disease state who has not been treated by the methods of the present invention. In one other embodiment, the treatment may include stabilization of serum creatinine levels in a subject where the serum creatinine levels observed in the subject are lower as compared to a subject with a similar disease state who has not been treated by the methods of the present invention. In another embodiment, the treatment may include stabilization of hematocrit (HCT) levels in a subject where the HCT levels observed in the subject are higher as compared to a subject with a similar disease state who has not been treated by the methods of the present invention. In another embodiment, the treatment may include stabilization of red blood cell (RBC) levels in a subject where the RBC levels observed in the subject are higher as compared to a subject with a similar disease state who has not been treated by the methods of the present invention. Those of ordinary skill in the art will appreciate that one or more additional indicators described herein or known in the art may be measured to determine the effective treatment of a kidney disease in the subject.

### Methods and Routes of Administration

The SRC+ cell populations and/or bioactive cell organoids of the present invention can be administered alone or in combination with other bioactive components. The SRC+ cell populations and/or organoids are suitable for injection or implantation of incorporated tissue engineering elements to the interior of solid organs to regenerate tissue.

In one aspect, the present invention provides methods of providing a bioactive cell organoid(s) described herein to a subject in need. In one embodiment, the source of the bioactive cell may be autologous or allogeneic, syngeneic (autogeneic or isogeneic), and any combination thereof, In instances where the source is not autologous, the methods may include the administration of an immunosuppressant agent. Suitable immunosuppressant drugs include, without limitation, azathioprine, cyclophosphamide, mizoribine, ciclosporin, tacrolimus hydrate, chlorambucil, lobenzarit disodium, auranofin, alprostadil, gusperimus hydrochloride, biosynsorb, muromonab, alefacept, pentostatin, daclizumab, siroiimus, mycophenolate mofetil, leflonomide, basiliximab, dornase α, bindarid, cladribine, pimecrolimus, ilodecakin, cedelizumab, efalizumab, everolimus, anisperimus, gavilimomab, faralimomab, clofarabine, rapamycin, siplizumab, saireito, LDP-03, CD4, SR-43551, SK&F-106615, IDEC-114, IDEC-131, FTY-720, TSK-204, LF-080299, A-86281, A-802715, GVH-313, HMR-1279, ZD-7349, IPL-423323, CBP-1011, MT-1345, CNI-1493, CBP-2011, J-695, UP-920, L-732531, ABX-RB2, AP-1903, IDPS, BMS-205820, BMS-224818, CTLA4-1g, ER-49890, ER-38925, !SAtx-247, RDP-58, PNU-156804, LIP-1082, TMC-95A, TV-4710, PTR-262-MG, and AGI-1096 (see U.S. Patent No. 7,563,822). Those of ordinary skill in the art will appreciate other suitable immunosuppressant drugs.

The treatment methods of the subject invention involve the delivery of SRC+ cell populations and/or organoids described herein. In one embodiment, direct administration of cells to the site of intended benefit is preferred.

A variety of means for administering organoids to subjects will, in view of this specification, be apparent to those of skill in the art. Such methods include, but are not limited to, intra-parenchymal injection, sub-capsular placement, trans-urethral catheterization, and intra-renal artery catheterization.

Cells can be inserted into a delivery device or vehicle, which facilitates introduction by injection or implantation into the subjects. In certain embodiments, the delivery vehicle can include natural materials. In certain other embodiments, the delivery vehicle can include synthetic materials. In one embodiment, the delivery vehicle provides a structure to mimic or appropriately fit into the organ's architecture. In other embodiments, the delivery vehicle is fluid-like in nature. Such delivery devices can include tubes, e.g., catheters, for injecting cells and fluids into the body of a recipient subject. In a preferred embodiment, the tubes additionally have a needle, e.g., a syringe, through which the cells of the invention can be introduced into the subject at a desired location. In some embodiments, mammalian kidney-derived cell populations are formulated for administration into a blood vessel via a catheter (where the term "catheter" is intended to include any of the various tube-like systems for delivery of substances to a blood vessel). Alternatively, the cells can be inserted into or onto a biomaterial or scaffold, including but not limited to textiles, such as weaves, knits, braids, meshes, and non-wovens, perforated films, sponges and foams, and beads, such as solid or porous beads, microparticles, nanoparticles, and the like (*e*.*g*., Cultispher-S gelatin beads - Sigma). The cells can be prepared for delivery in a variety of different forms. For example, the cells can be suspended in a solution or gel. Cells can be mixed with a pharmaceutically acceptable carrier or diluent in which the cells of the invention remain viable. Pharmaceutically acceptable carriers and diluents include saline, aqueous buffer solutions, solvents and/or dispersion media. The use of such carriers and diluents is well known in the art. The solution is preferably sterile and fluid, and will often be isotonic. Preferably, the solution is stable under the conditions of manufacture and storage and preserved against the contaminating action of microorganisms such as bacteria and fungi through the use of, for example, parabens, chlorobutanol, phenol, ascorbic acid, thimerosal, and the like. One of skill in the art will appreciate that the delivery vehicle used in the delivery of the cell populations and admixtures thereof of the instant invention can include combinations of the above-mentioned characteristics,

Modes of administration of the organoids containing and/or formed from isolated renal cell population(s), for example, the B2 cell population alone or admixed with B4' and/or B3, include, but are not limited to, intra-parenchyrnal injection, sub-capsular placement, or renal artery. Additional modes of administration to be used in accordance with the present invention include single or multiple injection(s) via direct laparotomy, via direct laparoscopy, transabdominal, or percutaneous. Still yet additional modes of administration to be used in accordance with the present invention include, for example, retrograde and ureteropelvic infusion. Surgical means of administration include one-step procedures such as, but not limited to, partial nephrectomy and construct implantation, partial nephrectomy, partial pyelectomy, vascularization with omentum ± peritoneum, multifocal biopsy needle tracks, cone or pyramidal, to cylinder, and renal pole-like replacement, as well as two-step procedures including, for example, organoid-internal bioreactor for replanting. In one embodiment, the organoids containing and/or formed from admixtures of cells are delivered via the same route at the same time. In another embodiment, each of the organoids are delivered separately to specific locations or via specific methodologies, either simultaneously or in a temporally-controlled manner, by one or more of the methods described herein.

The appropriate cell or organoid implantation dosage in humans can be determined from existing information relating to either the activity of the organoids, for example EPO production, or extrapolated from dosing studies conducted in preclinical studies. From *in vitro* culture and *in vivo* animal experiments, the amount of organoids can be quantified and used in calculating an appropriate dosage of implanted material. Additionally, the patient can be monitored to determine if additional implantation can be made or implanted material reduced accordingly.

One or more other components can be added to the organoids comprising and/or formed from cell populations and admixtures thereof of the instant invention, including selected extracellular matrix components, such as one or more types of collagen or hyaluronic acid known in the art, growth factors, and/or cytokines, including but not limited to VEGF, PDGF, TGFp, FGF, IGF, platelet-rich plasma and drugs.

Those of ordinary skill in the art will appreciate the various methods of administration suitable for the organoids described herein.

### Articles of Manufacture and Kits

The instant invention further includes kits comprising the polymeric matrices and scaffolds of the invention and related materials, and/or cell culture media and instructions for use. The instructions for use may contain, for example, instructions for culture of the cells in the formation of the SRC+ cell pouplations or organoids of the invention and/or administration of the SRC+ cell pouplations or organoids, In one embodiment, the present invention provides a kit comprising a scaffold as described herein and instructions. In yet another embodiment, the kit includes an agent for detection of marker expression, reagents for use of the agent, and instructions for use. This kit may be used for the purpose of determining the regenerative prognosis of a native kidney in a subject following the implantation or administration of an organoid(s) described herein. The kit may also be used to determine the biotherapeutic efficacy of an organoid(s) described herein.

Another embodiment of the invention is an article of manufacture containing organoids containing and/or formed from bioactive cells useful for treatment of subjects in need. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating a condition and may have a sterile access port (for example the container may be a solution bag or a vial having a stopper pierceable by an injection needle). The label or package insert indicates that the organoid is used for treating the particular condition. The label or package insert will further comprise instructions for administering the organoid to the patient. Articles of manufacture and kits comprising combinatorial therapies described herein are also contemplated. Package insert refers to instructions customarily included in commercial packages of therapeutic products that contain information about the indications, usage, dosage, administration, contraindications and/or warnings concerning the use of such therapeutic products. In one embodiment, the package insert indicates that the organoid(s) is used for treating a disease or disorder, such as, for example, a kidney disease or disorder. It may further include other materials desirable from a commercial and user standpoint, including other buffers, diluents, filters, needles, and syringes. Kits are also provided that are useful for various purposes, e.g., for assessment of regenerative outcome. Kits can be provided which contain detection agents for urine-derived vesicles and/or their contents, *e.g*., nucleic acids (such as miRNA), vesicles, exosomes, etc., as described herein. Detection agents include, without limitation, nucleic acid primers and probes, as well as antibodies for *in vitro* detection of the desired target. As with the article of manufacture, the kit comprises a container and a label or package insert on or associated with the container. The container holds a composition comprising at least one detection agent. Additional containers may be included that contain, e.g., diluents and buffers or control detection agents. The label or package insert may provide a description of the composition as well as instructions for the intended *in vitro,* prognostic, or diagnostic use.

The following examples are offered for illustrative purposes only, and are not intended to limit the scope of the present invention in any way.

### EXAMPLES

### Example 1 - Isolation of Selected Renal Cell +(SRC+) cell populations: isolation of endothelial cells from renal biopsy

The following method provides an example of endothelial cell isolation following enzymatic digestion using a previously adapted Collagenase/Dispase digestion protocol ^{2,3,5}. This method has been applied to diseased ZSF1 rat kidneys and to a kidney biopsy obtained from a hypertensive human ESRD patient on dialysis.

### Endothelial Cells (EC), vascular and lymphatic origin:

Digested kidney(s) using standard operating procedures for kidney cell isolation, as described *infra.,* are filtered through a 100µm *Steriflip* filter (*Millipore*). The remaining cell suspension is neutralized with DMEM containing 5% FBS and then washed by centrifugation at 300xg for 5 minutes. The cell pellet recovered through the 100 µm filter is re-suspended In fully supplemented EGM-2 growth medium (*Lonza*) and plated onto fibronectin coated dishes at a cell density of 25K/cm². The cultures are fed every 3 days. When the EC cultures are 80-90'% confluent, they are trypsinized and counted. The trypsinized cells are labeled with CD31 (PECAM) primary antibody and positively selected using *Miltenyi* anti-CD31 microbeads. The CD31⁺ sorted cells are washed, counted, plated and seeded at a density of 10K/cm² in fully supplemented EGM-2 medium. Endothelial cell morphology should be evident post twenty four hours and these cells can be expanded through multiple passages. The vascular and/or lymphatic composition may be analyzed using lineage-specific antibodies (e.g. VEGF3 -vascular capillary; LYVE1- lymphatic); the specific endothelial subpopulation can be further selected/sorted using the same cell surface phenotyping markers, using the *Miltenyi* micro-bead selection method.

After expansion and purification, the endothelial cell component of the NKA SRC can be enriched to a larger percentage (>30%) than the natural frequency as observed through buoyant density fractionation (<2%). In one embodiment, for controlling the active biological ingredients or composition of NKA, the purified EC can be combined at a selected frequency with the B2 and/or B2-B3-B4 fractions previously described from buoyant density gradient fractionation (e.g. 60% B2-B4 + 40% EC) prior to transplantation.

### Endothelial cell sorting using magnetic micro-beads:

Cells harvested from the fibronectin coated flasks in EGM2 medium are stained with mouse anti-human CD31 primary antibody at a concentration of 0.4µg/million cells/100µl in EGM2 medium w/o supplements for 20 minutes at 4ºC. After 20 minutes the cells are washed and re-suspended in 12mls of EGM2 medium w/o supplements and 200 µl of goat anti-mouse IgG1. Miltenyi microbeads are added at and incubated for an additional 20 minutes at 4ºC protected from light. After 20 minutes the cells are washed twice via centrifugation (5min @ 300xg) and re-suspended in 12mls [10 million cells/ml] and purified using a Miltenyi auto-MACS instrument (Double Positive Selection in Sensitive Mode program).

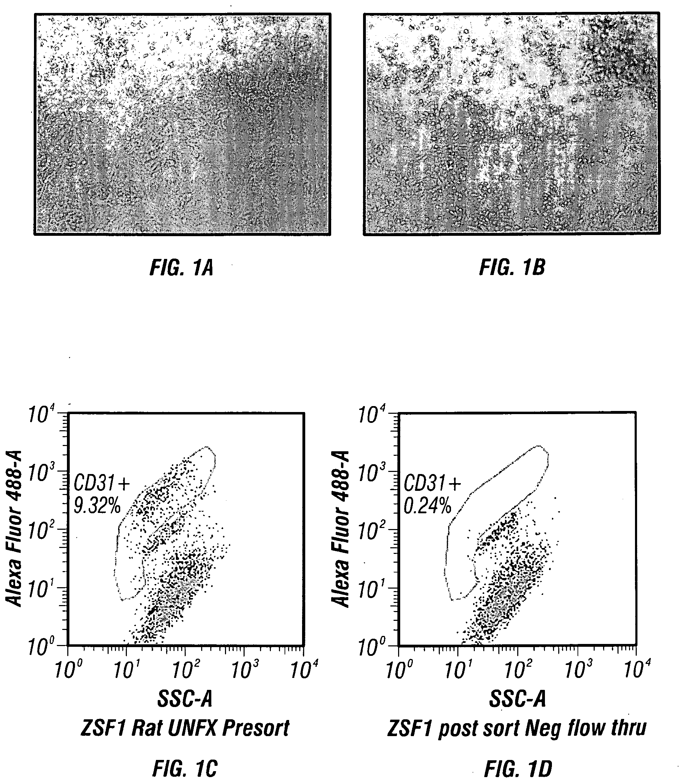

In the example described in FIG. 1, endothelial cells were selected (CD31+ selection) from primary ZSF1 cells. The 3.6 million cells recovered from this process were counted and sub-cultured onto two 1175 fibronectin coated flask at a concentration of 10K per cm2. The cells were cultured for four days at 21%02 and harvested at 85% confluency. Following this selection and culture period, the cells were counted and 9.5 million cells were recovered. The sample collected for phenotypic analysis by FACS was ^{~} 90% positive for CD31 (FIG. 1E, right FACS panel).

### Human CKD Endothelial Cell Isolation, Characterization and Expansion

### Human kidney cell culture:

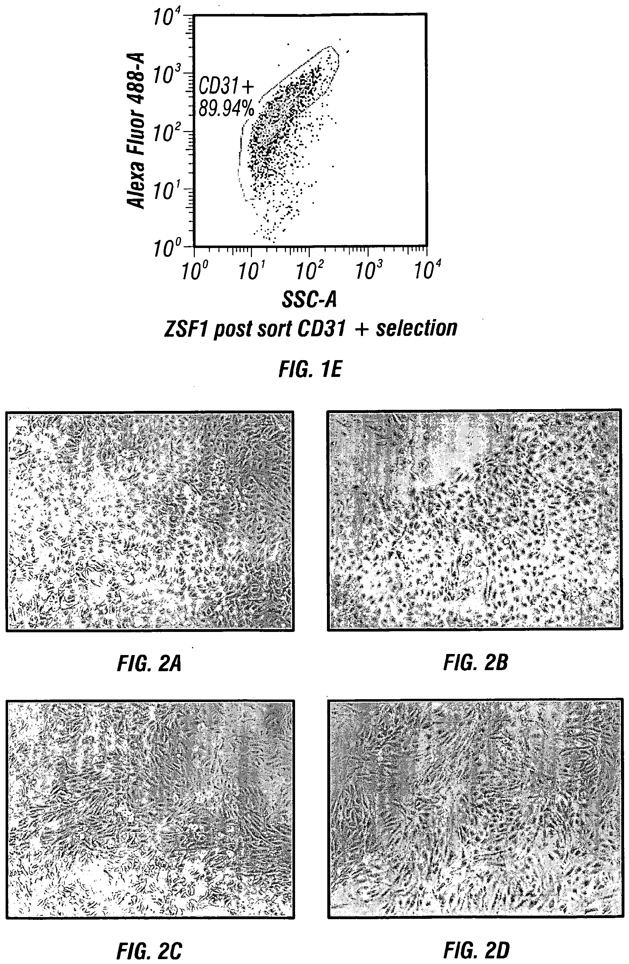

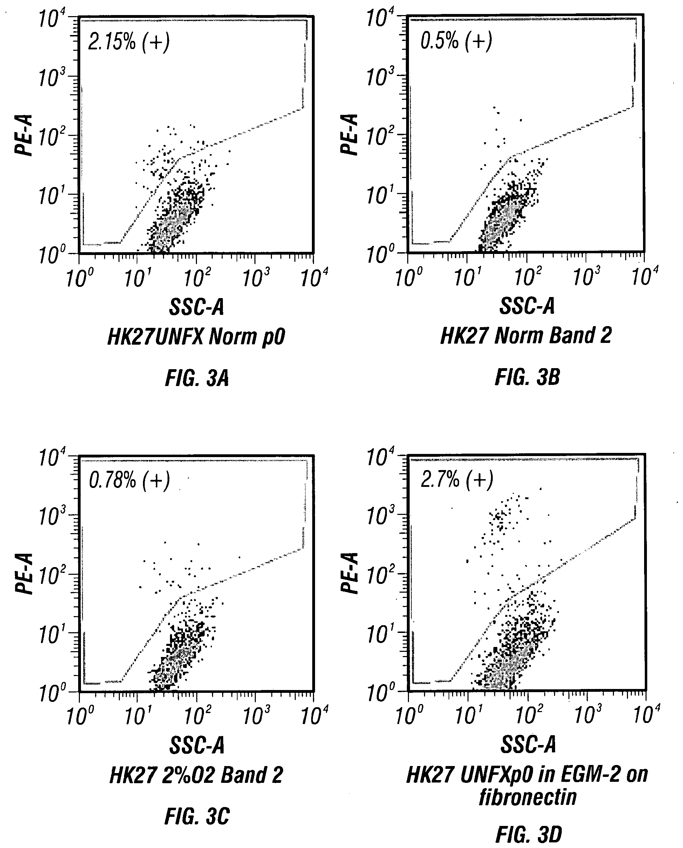

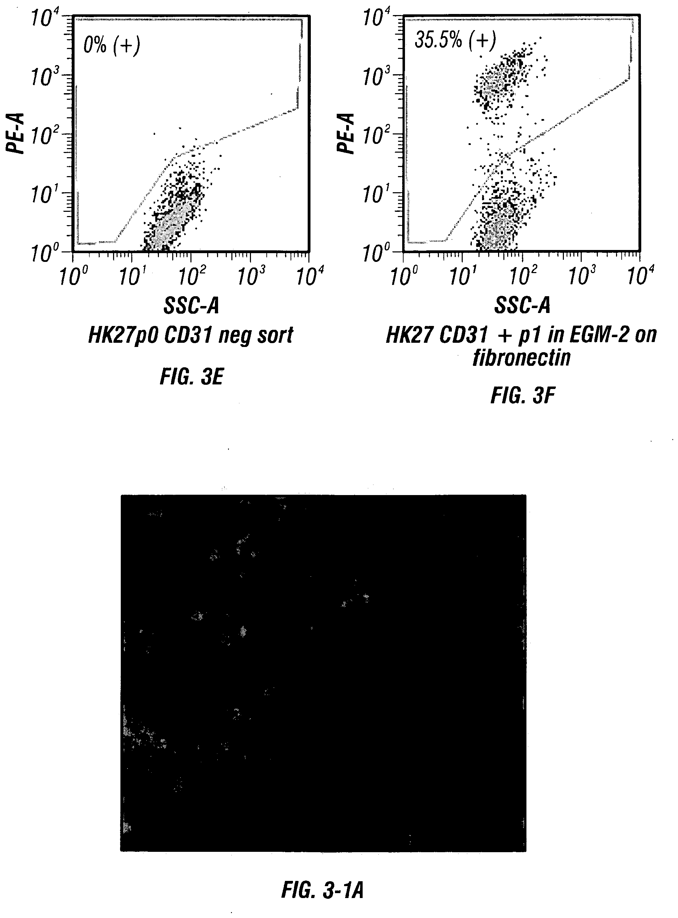

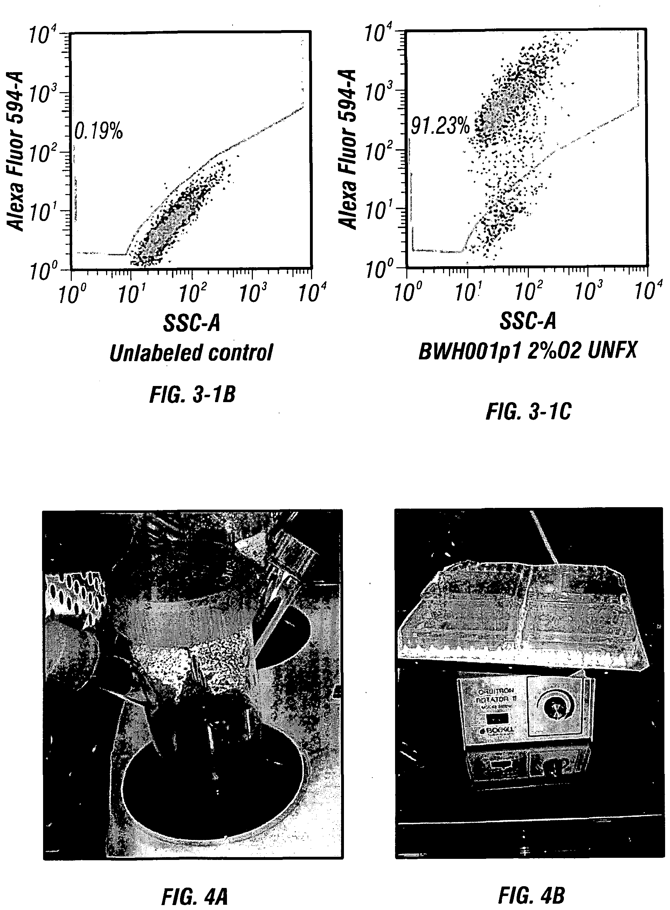

Human kidney cells (See Table 1 below for donor information) were isolated using standard operating procedures of human kidney primary cell enzymatic isolation and culture, as described *infra.* Briefly, cells were isolated and cultured at 25K/cm² in either T/C treated flask with standard KGM or on fibronectin coated flask with EGM2 fully supplemented medium. The cells were grown for three days in a 21% oxygen environment and then the medium was changed and the cultures were moved to a 2% oxygen environment for O/N exposure. After four days the cells were imaged to analyze culture morphology (FIG. 2A). They were then harvested and counted using standard methods (see HK027 Batch Record). A sample was collected and stained to determine the percentage of CD31⁺ endothelial cells (FIG. 2). Endothelial cells were sorted using a mouse anti-human CD31 primary antibody (BD biosciences) and magnetic microbeads (Miltenyi). CD31⁺ cells were then re-plated onto fibronectin coated flasks at a density of 10K/cm² and cultured an additional four days (FIG. 2, cell culture morphology). The cells were then counted and a sample was collected to determine the percentage positive endothelial cells using a mouse anti-human CD31 primary and FACS analysis (FIG. 3).

**Table 1. Human Kidney Donor Information:**

| **Sample ID** | **Cause of Death** | **BUN** | **sCrea** | **HCT** | **HB** | **Key Features** |
|---|---|---|---|---|---|---|
| HK027 | ICH | 72 | 11.1 | 42.8 | 14 | HTN for 20yrs, ESRD, on dialysis since 07' |

In conclusion, the initial culture of diseased ZSF1 cells on fibronectin yielded a 9% CD31⁺ culture (<2% from TC-treated). At p1, the 9% CD31⁺ fraction sub-cultured on fibronectin yielded ^{~} 90% CD31⁺ cells that expanded nearly 3-fold from p0 to p1. Initial culture of human CKD cells on either T/C treated or fibronectin coated flask yielded approximately the same percentage of CD31 positive cells (2.1% compared to 2.7%), As shown, it is possible to sub-culture the positively selected CD31 positive cells on fibronectin coated flasks in fully supplemented EGM2 medium and increase the percentage CD31 positive cells by 13 fold (35.5% from 2.7%) after passage 1.

### Isolation of SRC+ cell populations: Examples of the isolation of further bioactive cell types:

**A. Renal Epithelia:** Ongoing activities are evaluating the representation of the various epithelial cell compartments (parietal, proximal tubular, loop of Henle, distal tubular cells) in the fractions isolated through buoyant density gradients. More specifically, the lineage tracing studies provide direct and selective confirmation of Six2⁺ epithelial cell isolation; co-labeling of Six2 with a marker specific to a nephron epithelial compartment (See Tables 2-3) confirms cell-specific detection (See Table 4). Table 4 provides a limited panel of epithelial markers that characterizes p0 ZSF1 culture (combined fractions B2-B3-B4) as a mixture of proximal, distal and collecting duct cell types. Using the same cell surface detection antibodies listed in Tables 2-4, the enrichment of these specialized epithelial cell compartments through culture selection as previously described (culture conditions along with magnetic sorting) is contemplated.

**Table 2. Marker panel for tissue specific nephron staining**

| | **Proximal Tubule** | | | **Distal Tubule** | | | |
|---|---|---|---|---|---|---|---|
| **Antigen** | **Convoluted** | **Straight** | **Loop of Henle** | **Ascending Thick Limb** | **Macula Densa** | **Convoluted** | **Collecting Duct** |
| **Cyto keratin** | | | | | | | |
| CK18 | Pos | Pos | Pos | Pos | Pos | Pos | Pos |
| CK7 | Neg | Neg | Pos | Pos | Neg | Pos | Pos-ind |

| **Other** | | | | | | | |
|---|---|---|---|---|---|---|---|
| E-CAD | Neg | Neg | Pos | Pos | Pos | Pos | Pos |
| N-CAD | Pos | Pos | Neg | Neg | Neg | Neg | Neg |
| Aquaporin 1 | Pos | Pos | Neg | Neg | Neg | Neg | Neg |
| Aquaporin 2 | Neg | Neg | Neg | Neg | Neg | Neg | Pos |
| THP | Neg | Neg | Neg | Pos | Pos | Pos | Pos |
| **Lectins** | | | | | | | |
| LTA | Pos | Pos | Pos | Neg | | Neg | Med-ind |
| DBA | Neg | Neg | Pos | Pos | | Pos | Pos-ind |
| UEA | Neg | Neg | Neg | Neg | | Neg | Pos-ind |

| | | | | | | | |
|---|---|---|---|---|---|---|---|
| Pos= positive, Pos-ind = positive cell in selected population of cells, Med -ind = medulary collecting ducts stain pos | | | | | | | |

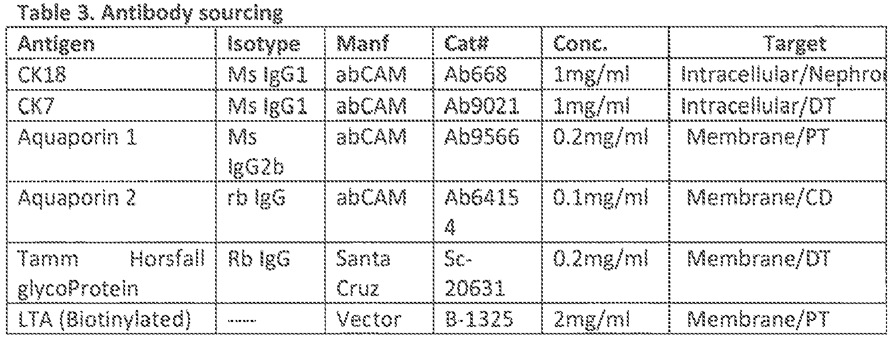

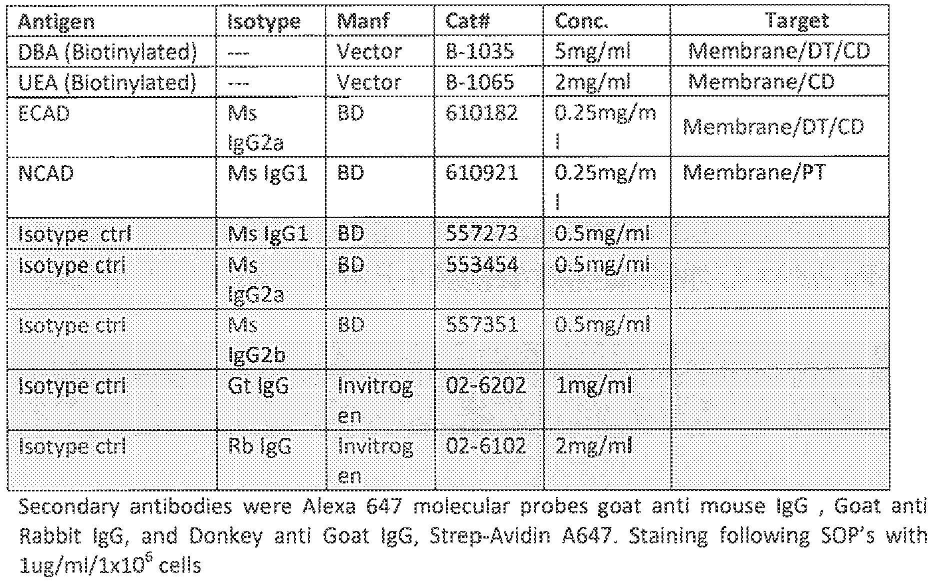

**Table 4. Preliminary ZSF1 p0 cell specific detection**

| **Marker** | **Compartment** | **ZSF1 p0 % positive** |
|---|---|---|
| AQP2 | Collecting duct | 42.36 |
| DBA | Distal Tubule | 56.93 |
| LTA | **Proximal/Loop/IMCD** | 39.62 |
| CK18 | Pan-epithelial | 68.39 |

**B. Glomerular Derived Cells (parietal epithelial and podocytes):** Anatomical exclusion of the pelvis improves the isolation of the glomerular fraction. Using standard operating procedure for kidney digest, described *infra.,* cells are Isolated and filtered through a 100µm *Steri-flip* filter (*Millipore*), and cell particles/clumps larger than 100µm (contains the glomerular fraction) are re-digested for additional 20minutes. The digested fraction is neutralized with DMEM medium containing 10% FBS and washed by centrifugation (300xg for 5min). The re-suspended cell pellet Is cultured in VRADD medium⁶ (DMEM/F12, 1uM All Trans Retinoic Acid (*Genzyme,* Cambridge, MA), 0.1um Dexamethasone (*Sigma-Aldrich*), 10-100nM Vitamin D (1,25(OH)2D3 to promote podocyte culture, or a parietal epithelial friendly serum free medium such as (50:50 DMEM/KSFM) fully supplemented without FBS, cultured on Collagen Type 1 coated T/C plates. Cells are seeded at a density of 25K/cm² and subculture until cell out-growth appears. Expand and sort outgrowth cells using primary antibody bound to *Miltenyi* micobeads at passage 1 using either PEC specific markers (such as but not limited to Claudin-1) or progenitor specific markers (e.g. CD146, CD117, SOX2, Oct 4A, CD24, CD133) or mesangial specific markers (such as but not limited to Smooth Muscle Actin, Vimentin, Myocardin, Calbindin) or glomerular capillary endothelial cells (e.g. VEFG3 or CD31 or LIVE1). When harvested at optimal cell yields and combined with the B2 component at a greater than natural frequency, a higher percentage of glomerular-derived cells can be applied to patients afflicted with glomerular disease.

**C. Collecting Duct Epithelial:** Anatomical exclusion of the cortex and medullary region of the kidney improves the isolation of cells located in or near the pelvis. Standard operating procedures for isolation and expansion and harvest of primary kidney cells for rodent, canine and human, as described *infra.,* is followed for isolation and expansion of collecting duct epithelial cells. Standard operating procedures for fractionating sub-populations of primary cultured kidney cells enrich for cells of the collecting ducts in cell fraction B1, are described herein *infra.* These cells contain the highest percentage of collecting duct epithelial cells based on makers such as Aquaporin 2 and *Dolichos Biflorus Agglutinin* (DBA). Alternatively, we have adapted a papilla/inter-medullary collecting duct (IMCD) culture protocol from Dr. Ben Humphreys (REGM media supplemented with EGF; unpublished data). In any of these scenarios, a cell fraction enriched for collecting duct epithelia can be used in combination with B2 component at higher than natural frequencies or they can be expanded using standard KGM or equivalent and used to target diseased etiologies associated with urine concentration and may better substantiate the active biological ingredients that could be applied to abnormalities and/or diseases of the renal pelvis (e.g. hydro-nephrosis, vaso-ureter obstruction).

### Example 2- Generation and characterization of Organolds from SRC and SRC+ populations Self-generated Organoid/Spheroid Formation

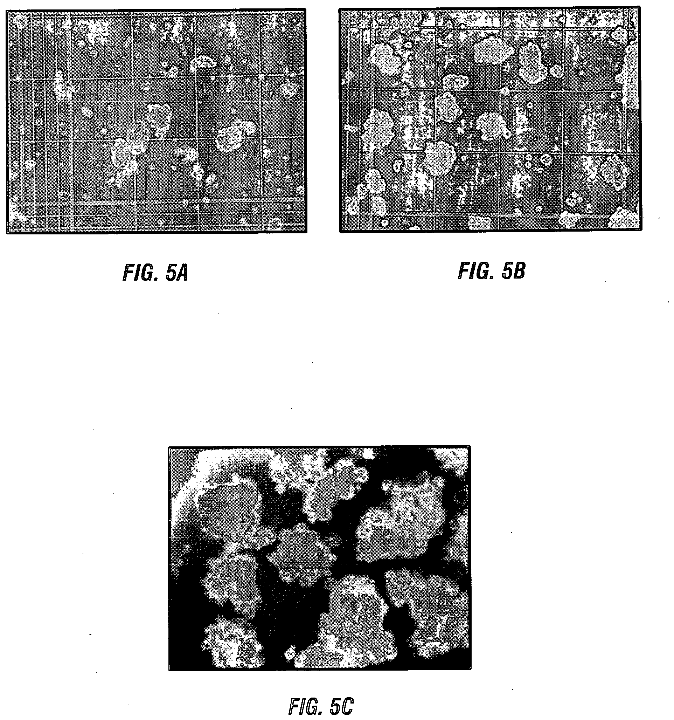

Organoids were generated following primary kidney culture, expansion and buoyant density gradient centrifugation to isolate SRC (standard operating procedures for generating HKA, as described *infra*)*.* Briefly, SRC were re-suspended in 100ml of renal cell growth medium at a concentration of [1×10⁶ cells/ml] in spinner flasks (Corning) for 24-48hrs on a magnetic stirrer set at 80rpm (FIG. 4). Cell organoids/spheroids consist of clusters of cells ranging in size from 50-125µM. Cell number per organoid can vary based on cell type and size prior spinner flask culture. Organoid/spheroids have been generated from both rat and human SRC and express a tubular epithelial phenotype (FIG. 5).

### Organoid function and Tubulogenesis

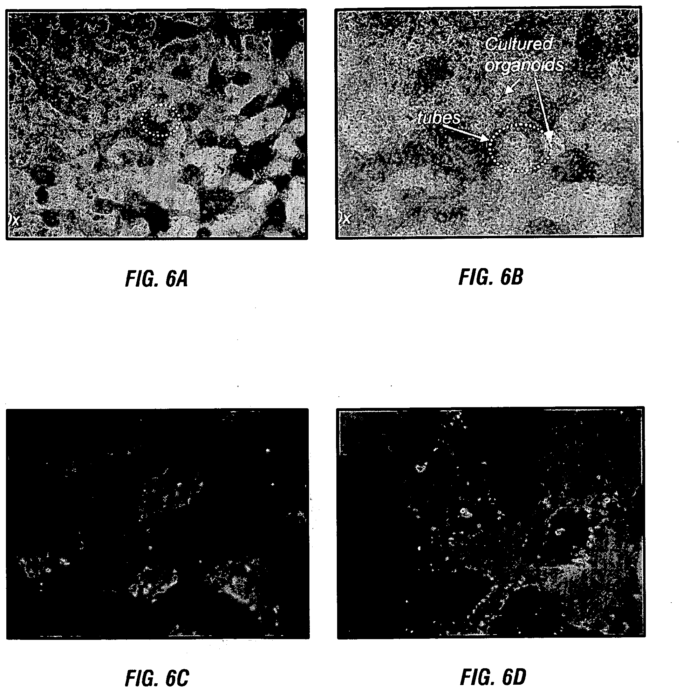

The ability of SRC to form tubes may be applied to assess potency of NKA. This assay was applied to the SRC organoids cultured in a 50:50 mixture of Collagen 1/Collagen IV gelatin in 3D. Upon immuno-fluorescent staining of the cultured organoids, the resultant tubes continue to express an epithelial phenotype (FIG. 6).

### Organoid Plus

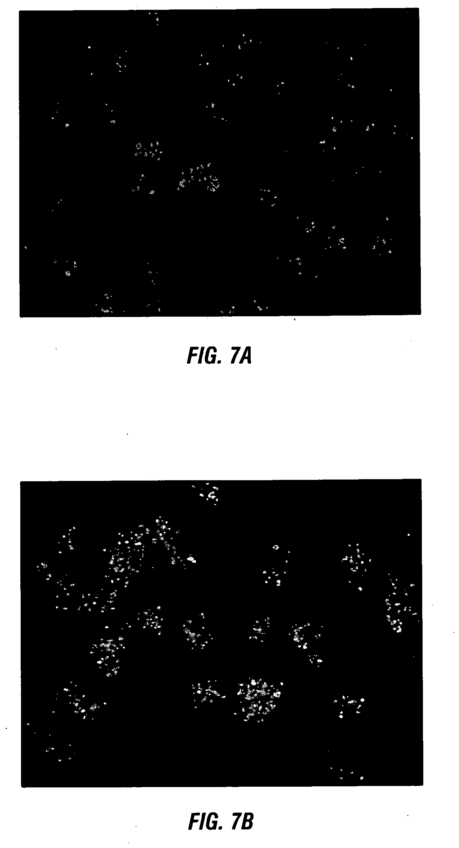

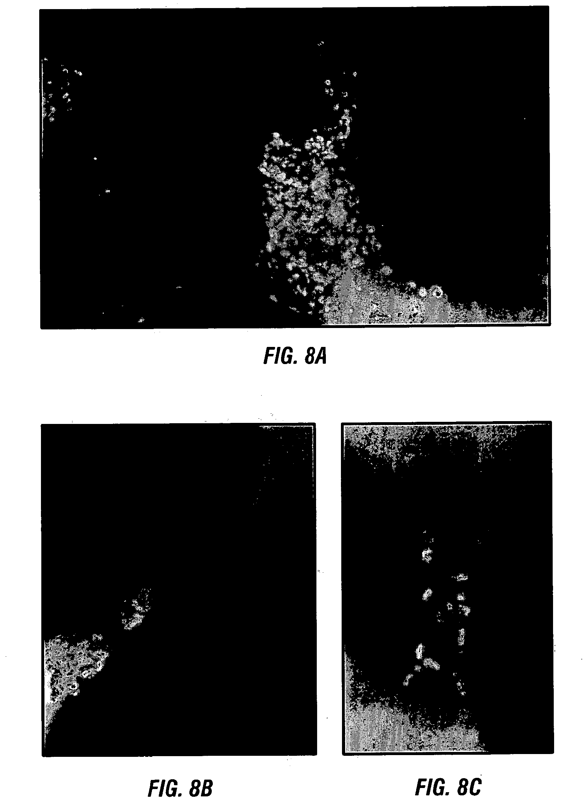

The ability of SRC to form self-generated spheroids may prove advantageous when applying combinations of other cell types. Tubulogenesis may be enhanced with the addition of a vascular or stem cell component. By adding a selective cell population in culture with the SRC population during the organoid formation period, a functional unit may be formed recapitulating key cell signaling pathways activated during regenerative outcomes. Examples of such cell-cell interactions include but are not limited to epithelial-mesenchymal signaling events known to be pivotal during organogenesis (see Basu & Ludlow 2012, Developmental engineering the kidney-leveraging morphogenic principles for renal regeneration. Birth Defects Research Part C 96:30-38*).* While SRC are generated using standard procedures, an endothelial cell line (HuVEC) was used as an example of an organoid(+) combination. Eighty million SRC were labeled with a membrane dye (invitrogen DIL red label) and added to 20 million HuVEC, labeled with a different color membrane dye (Invitrogen DiO green label) in 100ml of RCGM medium in 125ml spinner flasks at 80rpm for 48hrs (FIG. 7). An SRC organoid population alone was also started as control. Upon formation of organoids, tubulogenesis assays were set-up in-vitro within Col I/IV 3D gels to ensure the ability to form tubes with and without potential concomitant vascularization (FIG. 8).

### Example 3 - Characterization/biodistribution of SRC organoids in rodent models of kidney disease

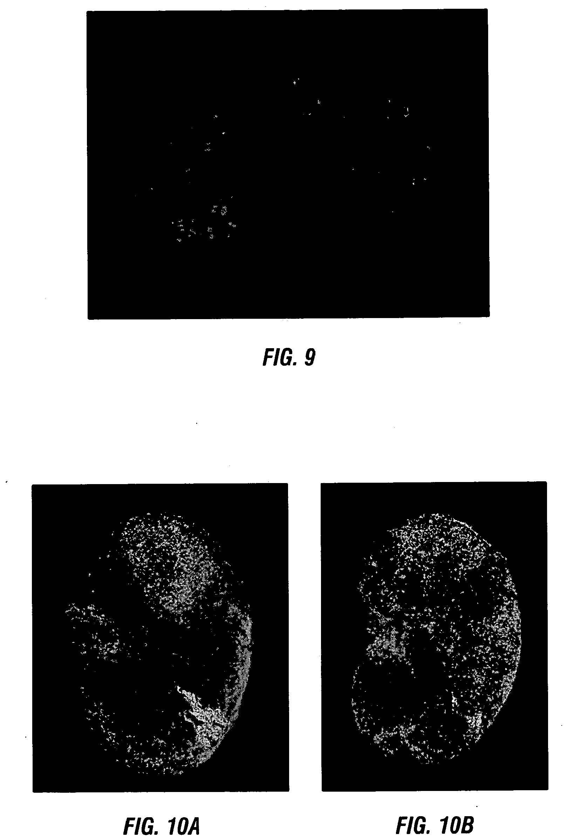

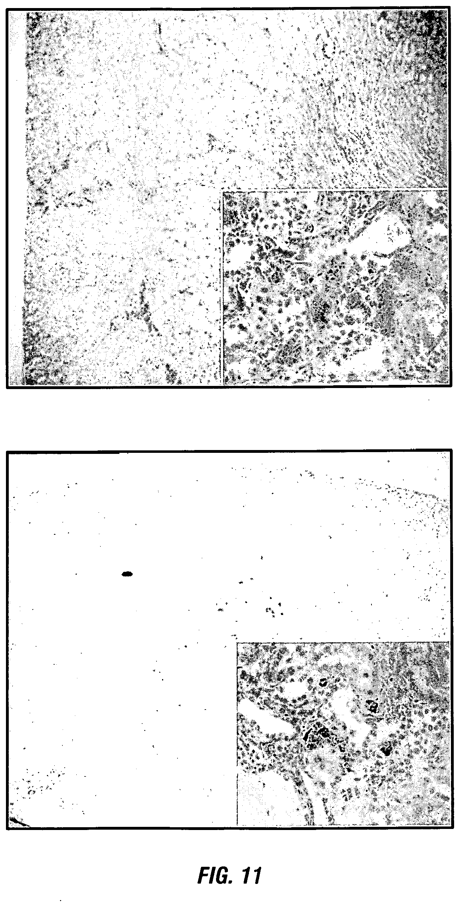

ZSF1 acute study evaluating bio-distribution and cell retention of SPIO labeled organoids over a 48 hr period. Six 40+ week old ZSF1 rats were injected into the parenchyma with 2.5×10⁶ SPIO Rhodamine labeled cells (representing approximately 25000 self-generated organoids in PBS) at a concentration of 50×10⁶/ml in left caudal pole. (Figure 9). Three animals were harvested at intervals of 24 and 48 hrs post implantation. Kidneys were evaluated by MRI and histologically using Prussian blue and H&E staining method for cell retention and bio-distribution (Figure 10 & 11).

### RESULTS

Organoids were readily labeled and traced following targeted delivery. Organoid treatment was well tolerated with no morphological alterations observed in the tubular or glomerular compartments. Multifocal Clusters of epitheliod cells (staining positive by Prussian blue) were frequently observed in the renal cortex of left kidney (intra/inter tubular) at 24 hours post injection and to a lesser extent after 48 hours.

### Example 4 - In vivo studies to demonstrate therapeutic efficacy of SRC+ and SRC/SRC+ derived organoids: CKD (5/6 nephrectomy) immune-compromised rodent studies

Human-derived SRC+ and SRC/SRC+ organoids were evaluated for therapeutic efficiency using the S/6-nephrectomy model of chronic kidney disease in immune-compromised rodents (NIHRNU (nude) rats; athymic rats). Establishment of the disease state, treatment intervention modalities and clinical evaluation of therapeutic response was as previously described (Genheimer et al., 2012. Molecular characterization of the regenerative response induced by intrarenal transplantation of selected renal cells in rodent model of chronic kidney disease. Cells Tissues Organs 196: 374-84).

**Cell isolation and selection:** Human renal cells were isolated, expanded from biopsy as described in Presnell (2011). Cells were sub-cultured and cryopreserved after passage 2 in cryopreservation buffer (80% HTS/10%DMSO/10% FBS) at a concentration of (20×10⁶ cells/ml/vial) using a freezing rate of -1°C/min down to -80°C and then transferred to the liquid nitrogen freezer for longer storage. Following cryopreservation, the cells were quickly thawed at 37°C to assess recovery and viability using standard Trypan blue exclusion method. Cells were then seeded onto tissue culture vessels at 3000 cells/cm² and cultured for 4 days under normoxic conditions (21%O₂/5% CO₂/37°C) in renal cell growth medium. After 4 days the culture medium was changed and the cultures were incubated overnight in a lower oxygen environment (2%O₂/5% CO₂/37°C) prior to cell harvest and selection of SRC by density gradient separation. Human Umbilical Vein Endothelial Cells (HUVEC) cultures (Lonza 2389, CAT# CC2517) were expanded from early passage following cryopreservation until passage 3 in EGM2 fully supplemented medium. Cells were harvested and viability was assessed using standard Trypan blue exclusion method.

**Organoid Formation:** Organoid cultures were established by 1) resuspending human SRC and HUVEC's to a concentration of 2×10⁶ cells/ml In their respective mediums 2) combining equal volumes of each cell preparation 25mls (50×10⁶ cells/ml) into a 125ml spinner flask (Corning). To the combined culture, 25mls of each medium was added to the flasks for a final cell concentration of 1×10⁶ cells/ml in 100mls. The flask was placed on a magnetic stirrer in the incubator at a speed of 80RPM and cultured for 24 hrs.

**Test Articles:** The organoid dose was adjusted such that approximately (2- 5 ×10⁶ cells/50µl) were to be administered per rat kidney. Organoid numbers were adjusted to the concentration above in a larger volume of 0.5mL of DPBS to account for any loss during shipping. An extra tube was sent as replacement. The doses were sent via FEDEX to the implantation facility in two 0.5ml microcentrifuge tubes using a CREDO cube shipper at 4-8°C.

**Test Article Delivery:** The remnant left kidney was accessed through left flank incision. Prior to delivery, organoids were gently resuspended by tapping or flicking the base of the tube (no vortexing). The organoids were aspirated with an 18G blunt tip needle into the syringe. The needle was then changed to a 23G cutting needle for delivery. Targeted delivery was carried out by injecting 50µl into the cortico-medullary region of the kidney. Untreated control animals remained untreated and underwent no procedure.

**RKM Model Procedures:** Male NIHRNU rats 8-12 weeks old (approx. 200 g average weight) underwent 2 step nephrectomy procedures as follows: For each individual animal the right kidney was removed and weighed, recovery allowed for 1 week, followed by resection of tissue from caudal and cranial poles of left kidney which was also weighed. A 2-3 week recovery and acclimation period followed prior to the beginning of the studies. Following the acclimation period, blood and urine were collected for 2 consecutive weeks and analyzed. Subsequent sampling was performed every 2 weeks thereafter. All rodents were fed a commercially available feed, and water was provided *ad libitum.*

Animals were monitored post-nephrectomy with some evidence of increasing urine protein/urine protein creatinine (UPC) and were treated with human organoids at 12 weeks post-nephrectomy. Table 5 below provides an overview of the study.

**Table 5: Study Design**

| **Nephrectomy Batch** | **DESCRIPTION (Treatment)** | **Assessments** | |
|---|---|---|---|
| **Animals (59% KMR)** | Organoid Prototype (n=4) | **Daily** | |
| | | | • **Survival** |
| | | **Bi-Weekly** | |
| | | | • Body weights |
| Tx 12 weeks post nephrectomy | Untreated (n=8) | | • Serology and hematology |
| | | | • Urinalysis Panel |
| **Term of Observation** 204 days post Nephrectomy 119 days post-Tx | | **Necropsy** | |
| | | | • Gross pathology observations |
| | | | • Abdomen opened and whole animal fixed if unscheduled death |

| | | | |
|---|---|---|---|
| **Measurements:** Body weights (g) were collected on a biweekly basis). Serum biochemical and hematological evaluations were conducted for the duration of study on a bi-weekly basis. Urinalysis was performed biweekly At scheduled necropsy for study animals gross observations were made, the remnant kidney excised and fixed, and animal remains fixed in formalin, All animals and tissues were collected as needed, weighed, measured and prepared for histological processing. Methods used In histological evaluations of kidney were performed. KMR is kidney mass reduction. | | | |

### RESULTS:

The efficacy of human NKO was demonstrated in a renal insufficiency model in athymic rats.

### RKM Model Overview

Disease progression within the athymic KMR model was consistent with previously described nephrectomy models and CKD-related sequelae including disruption of kidney protein handling, azotemia, hypercholesterolemia, anemia, and developing uremia. The outcome of NIHRNU Nephrectomy model in regard to disease state achieved was sensitive to the amount of kidney tissue resected during mass reduction and the animals utilized here demonstrated a slowly progressing early stage disease state based upon clinical pathology monitoring and terminal histological evaluations.

### NKO Prototype Effect on In-Life Clinical Pathology

Clinical pathology measures relevant to kidney function were examined pretreatment and compared in a paired fashion to measures taken prior to end of study. From this comparison, as summarized in the table below, there were changes consistent with disease progression over the 4 month study in untreated control animals. Significant differences were noted in paired BUN, Hct, Hgb, RBC, WBC, sCho!, sProt, sAlb, uPro, UPC, and spGrav. Changes in these markers are typical of early stage CKD and indicative of decreases in renal function that have yet to result in acute azotemia, end stage loss of tubule filtration/concentration and phosphotemia. In animals treated with NKO, changes in BUN, Hct, Hgb, RBC, WBC, and spGrav were not significant as would be predicted based upon untreated control animal outcomes (see Table 6 below).

**Table 6. Treatment Group Clinical Measures Pre- and Post-Treatment.**

| | | | **Time 0** | | **Endo Study** | | **Significant** | **Pasred t-test** |
|---|---|---|---|---|---|---|---|---|
| **Measure** | | **Group** | **Average** | **SD** | **Average** | **SD** | **Change** | **p-value** |
| **sCre** | | Control | 0.48 | 0.07 | 0.51 | 0.21 | | 0.598 |
| | | Organoid | 0.43 | 0.05 | 0.58 | 0.29 | | 0.298 |
| **BUN** | | Control | 32.4 | 4.2 | 47.1 | 19.5 | YES | 0.045 |
| | | Organoid | 31.8 | 5.7 | 56.5 | 27.8 | | 0,09 |
| **Hct** | | Control | 46.56 | 2.48 | 39.99 | 2.71 | Yes | <0.0001 |
| | | Organoid | 42.48 | 1.38 | 39.1 | 3.71 | | 0.26 |

| | | **Time 0** | | | **End of Study** | | **Significant** | **Paired t-test** |
|---|---|---|---|---|---|---|---|---|
| **Measure** | **Group** | **Average** | **SD** | | **Average** | **SD** | **Change** | **p-value** |
| **Hgb** | Control | 14.85 | 0.92 | | 12.93 | 0.83 | Yes | 0.0005 |
| | Organoid | 14.18 | 0.25 | | 12.63 | 1.05 | | 0.0518 |
| **RBC** | Control | 8.93 | 0.49 | | 7.88 | 0.59 | Yes | 0.0007 |
| | Organoid | 8.51 | 0.17 | | 7.76 | 0.68 | | 0.124 |
| **WBC** | Control | 6.13 | 0.6 | | 8.74 | 2.15 | Yes | 0.016 |
| | Organoid | 6.93 | 0.43 | | 7.4 | 0.76 | | 0.406 |
| **sPhos** | Control | 7.28 | 0.88 | | 6.38 | 1.1 | | 0.15 |
| | Organoid | 6.75 | 0.51 | | 7.7 | 1.87 | | 0.48 |
| **sChol** | Control | 89.1 | 9.6 | | 139 | 47.4 | Yes | 0.01 |
| | Organoid | 77.8 | 4.1 | | 142.5 | 15.4 | Yes | 0.005 |
| **sProt** | Control | 5.99 | 0.24 | | 5.28 | 0.28 | Yes | 0.001 |
| | Organoid | 5.83 | 0.26 | | 5.38 | 0.1 | Yes | 0.04 |
| **sAlb** | Control | 3.15 | 0.11 | | 2.64 | 0.23 | Yes | 0.0003 |
| | Organoid | 2.95 | 0.06 | | 2.58 | 0.05 | Yes | 0.0004 |
| **uPro** | Control | 371.8 | 211.5 | | 625.6 | 182.7 | Yes | 0.007 |
| | Organoid | 252 | 127.7 | | 674.9 | 33.63 | Yes | 0.008 |
| **UPC** | Control | 4.47 | 2.08 | | 11.25 | 3.65 | Yes | <0.0001 |
| | Organoid | 4.39 | 0.95 | | 11.41 | 0.66 | Yes | 0.01 |
| **uCre** | Control | 83.4 | 22.9 | | 56.3 | 13.8 | | 0.06 |
| | Organoid | 55.75 | 17.86 | | 58.3 | 3.5 | | 0.87 |
| **uNa** | Control | 134.38 | 52.63 | | 61.13 | 12.88 | Yes | 0.005 |
| | Organoid | 110.25 | 34.79 | | 50.67 | 6.74 | | 0.116 |
| **uK** | Control | 116 | 35.66 | | 84.75 | 5.24 | | 0.104 |
| | Organoid | 79.5 | 22.75 | | 87.67 | 8.56 | | 0.549 |
| **uChlor** | Control | 163.88 | 20.7 | | 86.75 | 16.09 | Yes | 0.01 |
| | Organoid | 133.25 | 29.28 | | 79.33 | 7.02 | | 0.24 |
| **spGrav** | Control | 1.034 | 0.009 | | 1.025 | 0.003 | Yes | 0.02 |
| | Organoid | 1.027 | 0.009 | | 1.026 | 0.005 | | 0.73 |
| | | | | | | | | |

| Measure | Represents | | | Association with CKD | | | | |
|---|---|---|---|---|---|---|---|---|
| BUN | urea handling | | | decreased eGFR, tubular dysfunction | | | | |
| HCt | measures of anemia | | | early indicator of decreased kidney function; EPO dysfunction | | | | |
| Hgb | | | | | | | | |
| RBC | | | | | | | | |
| WBC* | inflammation | | | associated with increased risk for CKD progression | | | | |
| spGrav | urine concentration | | | decrease indicative of impaired tubule function | | | | |

| | | | | | | | | |
|---|---|---|---|---|---|---|---|---|
| *the WBC here would not reflect a T-cell mediated response as these animals are athymic | | | | | | | | |

### Histological Overview of Kidneys from Model

Histopathology demonstrated that control animals had progressed to mild, developing nephropathy by terminal sacrifice.

Injury-related findings were primarily tubular in nature with atrophy, dilation, and proteinaceous casts usually mild in severity. Tubular basophilia and fibrosis were often minimal and interstitial inflammation was typically minimal or absent. Glomerular changes were minimal in severity.

Capsular fibrosis/inflammation was typically only minimal in severity and was characterized by little inflammatory cellular content; the reaction may have been diminished by the incomplete immune system in the athymic nude rat. Mineralization and lymphocytic infiltration were of low incidence and severity in Batch 1 animals. Occasional focal mineralization, common in laboratory rats, was observed.

There were no significant differences noted between untreated and NKO treated groups.

**Table 7**

| Measure | Normal | Untreated | NKO Treated |
|---|---|---|---|
| Tubulo-lnterstital Injury | 0.0 + 0.1 | 1.4 ± 0.4 | 1.8 ± 0.4 |
| Glomerular Injury | 0.0 | 0.8 ± 0.3 | 1.2 ± 0.5 |

### NKO Effect on Survival

All animals (59% KMR) survived until end of study (119 days post-Tx and 204 days post-nephrectomy) therefore no discernable survival benefit of NKO treatment was detected.

### Engraftment of Human NKO Detected with Staining Methods at EOS

As this model allows xenogeneic application of a human product, tracking of human cells within the rat kidney was possible through use of human cell identifying antibodies. Staining for human HLA1 within the background of the RKM rat kidney identified cells 4 months post-treatment, see Figure 12. Identification of staining considered consistent with engraftment of human cells was confirmed by two independent reviewers. Typically 1 or 2 cells were identified in one of four sections stained for each animal and were incorporated into tubules, although one cluster was identified within the interstitium.

### CONCLUSIONS:

- Organoid (NKO) delivery mitigated significant changes associated with kidney disease progression in control animals and included measures effected by syngeneic/autologous NKA deliveries to disease models.
- Effected measures were consistent with those observed with syngeneic/autologous NKA deliveries to disease models - measures of anemia (Hct, Hgb, RBC), inflammation (WBC), urine concentration (spGrav) and possibly azotemia (BUN).
- Human cells in very low numbers were detected approximately 4 months post-treatment with NKO prototype.
- Nephrectomy of nude rats (59% KMR, average) resulted in a state of early stage and chronically progressive renal insufficiency characterized by developing anemia, proteinuria, dyslipidemia and possible indications of early stage azotemia.
- All animals survived until end of study.
- Procedures utilized to deliver human organoids to the RKM model were well tolerated.

### Example 5- Isolation & Characterization of Bioresponsive Renal Cells

A case of idiopathic progressive chronic kidney disease (CKD) with anemia in an adult male swine (*Sus scrofa*) provided fresh diseased kidney tissue for the assessment of cellular composition and characterization with direct comparison to age-matched normal swine kidney tissue. Histological examination of the kidney tissue at the time of harvest confirmed renal disease characterized by severe diffuse chronic interstitial fibrosis and crescentic glomerulonephritis with multifocal fibrosis, Clinical chemistry confirmed azotemia (elevation of blood urea nitrogen and serum creatinine), and mild anemia (mild reduction in hematocrit and depressed hemoglobin levels). Cells were isolated, expanded, and characterized from both diseased and normal kidney tissue. As shown in Figure 1 of Presnell et al. WO/2010/056328 (incorporated herein by reference in its entirety), a Gomori's Trichrome stain highlighs the fibrosis (blue staining indicated by arrows) in the diseased kidney tissue compared to the normal kidney tissue. Functional tubular cells, expressing cubulin:megalin and capable of receptor-mediated albumin transport, were propagated from both normal and diseased kidney tissue. Erythropoietin (EPO)-expressing cells were also present in the cultures and were retained through multiple passages and freeze/thaw cycles. Furthermore, molecular analyses confirmed that the EPO-expressing cells from both normal and diseased tissue responded to hypoxic conditions *in vitro* with HIF1α-driven induction of EPO and other hypoxia-regulated gene targets, including vEGF, Cells were isolated from the porcine kidney tissue via enzymatic digestion with collagenase + dispase, and were also isolated in separate experiments by performing simple mechanical digestion and explant culture. At passage two, explant-derived cell cultures containing epo-expressing cells were subjected to both atmospheric (21%) and varying hypoxic (<5%) culture conditions to determine whether exposure to hypoxia culminated in upregulation of EPO gene expression. As noted with rodent cultures (see Example 3), the normal pig displayed oxygen-dependent expression and regulation of the EPO gene. Surprisingly, despite the uremic / anemic state of the CKD pig (Hematocrit <34, Creatinine >9,0) EPO expressing cells were easily isolated and propagated from the tissue and expression of the EPO gene remained hypoxia regulated, as shown in Figure 2 of Presnell et al. WO/2010/056328 (incorporated herein by reference in its entirety). As shown in Figure 3 of Presnell et al. WO/2010/056328 (incorporated herein by reference in its entirety), cells in the propagated cultures demonstrated the ability to self-organize into tubule-like structures. As shown in Figure 4 of Presnell et al. WO/2010/056328 (incorporated herein by reference in its entirety), the presence of functional tubular cells in the culture (at passage 3) was confirmed by observing receptor-mediated uptake of FiTC-conjugated Albumin by the cultured cells. The green dots (indicated by thin white arrows) represent endocytosed fluorescein-conjugated albumin which is mediated by tubular cell-specific receptors, Megalin and Cubilin, indicating protein reabosroption by functional tubular cells. The blue staining (indicated by thick white arrows) is Hoescht-stained nuclei. Taken together, these data suggest that functional tubular and endocrine cells can be isolated and propagated from porcine renal tissues, even in renal tissues that have been severely compromised with CKD. Furthermore, these findings support the advancement of autologous cell-based therapeutic products for the treatment of CKD.

in addition, EPO-producing cells were isolated enzymatically from normal adult human kidney (as described above in Example 1). As shown in Figure 5 of Presnell et al. WO/2010/056328 (incorporated herein by reference in its entirety), the isolation procedure resulted in more relative EPO expression after isolation than in the initial tissue. As shown in Figure 6 of Presnell et al. WO/2010/056328 (incorporated herein by reference in its entirety), it is possible to maintain the human EPO producing cells in culture with retention of EPO gene expression. Human cells were cultured/propagated on plain tissue-culture treated plastic or plastic that had been coated with some extracellular matrix, such as, for instance, fibronectin or collagen, and all were found to support EPO expression over time.

### Example 6 - Isolation & enrichment of specific bioreactive renal cells

Kidney cell isolation: Briefly, batches of 10, 2-week-old male Lewis rat kidneys were obtained from a commercial supplier (Hilltop Lab Animals Inc.) and shipped overnight in Viaspan preservation medium at a temperature around 4°C. All steps described herein were carried out in a biological safety cabinet (BSC) to preserve sterility. The kidneys were washed in Hank's balanced salt solution (HBSS) 3 times to rinse out the Viaspan preservation medium. After the third wash the remaining kidney capsules were removed as well as any remaining stramaltissue. The major calyx was also removed using micro dissection techniques. The kidneys were then finely minced into a slurry using a sterile scalpel. The slurry was then transferred into a 50ml conical centrifuge tube and weighed. A small sample was collected for RNA and placed into an RNAse-free sterile 1.5ml micro-centrifuge tube and snap frozen in liquid nitrogen. Once frozen, it was then transferred to the -80 degree freezer until analysis. The tissue weight of 10 juvenile kidneys equaled approximately 1 gram. Based on the weight of the batch, the digestion medium was adjusted to deliver 20mls of digestion medium per 1 gram of tissue. Digestion buffer for this procedure contained 4 Units of Dispase 1(Stem Cell Tech) in HBSS, 300Units/ml of Collagenase type IV (Worthington) with SmM CaCl₂ (Sigma).

The appropriate volume of pre-warmed digestion buffer was added to the tube, which was then sealed and placed on a rocker in a 37°C incubator for 20 minutes. This first digestion step removes many red blood cells and enhances the digestion of the remaining tissue. After 20 minutes, the tube was removed and placed in the BSC. The tissue was allowed to settle at the bottom of the tube and then the supernatant was removed. The remaining tissue was then supplemented with fresh digestion buffer equaling the starting volume. The tube was again placed on a rocker in a 37°C incubator for an additional 30 minutes.

After 30 minutes the digestion mixture was pipetted through a 70µm cell strainer (BD Falcon) into an equal volume of neutralization buffer (DMEM w/ 10% FBS) to stop the digestion reaction. The cell suspension was then washed by centrifugation at 300xg for 5 min. After centrifugation, the pellet was then re-suspended in 20mls KSFM medium and a sample acquired for cell counting and viability assessment using trypan blue exclusion, Once the cell count was calculated, 1 million cells were collected for RNA, washed in PBS, and snap frozen in liquid nitrogen. The remaining cell suspension was brought up to 50mls with KSFM medium and washed again by centrifugation at 300xg for 5 minutes. After washing, the cell pellet was re-suspended in a concentration of 15 million cells per ml of KSFM,

Five milliliters of kidney cell suspension were then added to 5mls of 30% (w/v) Optiprep^{•} in 15ml conical centrifuge tubes (BD Falcon) and mixed by inversion 6 times. This formed a final mixture of 15% (w/v) of Optiprep^{•}, Post inversion, tubes were carefully layered with 1 mL PBS. The tubes were centrifuged at 800 x g for 15 minutes without brake. After centrifugation, the tubes were removed and a cell band was formed at the top of the mixing gradient. There was also a pellet containing red blood cells, dead cells, and a small population of live cells that included some small less granular cells, some epo-producing cells, some tubular cells, and some endothelial cells. The band was carefully removed using a pipette and transferred to another 15ml conical tube. The gradient medium was removed by aspiration and the pellet was collected by re-suspension in 1 ml KSFM. The band cells and pellet cells were then recombined and re-suspended in at least 3 dilutions of the collected band volume using KSFM and washed by centrifugation at 300xg for 5 minutes. Post washing, the cells were re-suspended in 20mls of KSFM and a sample for cell counting was collected. Once the cell count was calculated using trypan blue exclusion, 1 million cells were collected for an RNA sample, washed in PBS, and snap frozen in liquid nitrogen.

Pre-Culture 'Clean-up' to enhance viability and culture performance of Specific Bioactive Renal Cells Using Density Gradient Separation: To yield a clean, viable population of cells for culture, a cell suspension was first generated as described above in "Kidney Cell isolation". As an optional step and as a means of cleaning up the initial preparation, up to 100 million total cells, suspended in sterile isotonic buffer were mixed thoroughly 1:1 with an equal volume of 30% Optiprep^{®} prepared at room temperature from stock 60% (w/v) iodixanol (thus yielding a final 15% w/v Optiprep solution) and mixed thoroughly by inversion six times. After mixing, 1ml PBS buffer was carefully layered on top of the mixed cell suspension. The gradient tubes were then carefully loaded into the centrifuge, ensuring appropriate balance. The gradient tubes were centrifuged at 800 x g for 15 minutes at 25°C without brake. The cleaned-up cell population (containing viable and functional collecting duct, tubular, endocrine, glomerular, and vascular cells) segmented between 6% and 8% (w/v) Optiprep^{®}, corresponding to a density between 1.025 - 1.045 g/mL. Other cells and debris pelleted to the bottom of the tube.

Kidney Cell Culture: The combined cell band and pellet were then plated in tissue culture treated triple flasks (Nunc T500) or equivalent at a cell concentration of 30,000 cells per cm2 in 150m!s of a 50:50 mixture of DMEM(high glucose)/KSFM containing 5% (v/v)FBS, 2.51µg EGF, 25mg BPE, 1X ITS (insulin/transferrin/sodium selenite medium supplement) with antibiotic/antimycotic. The cells were cultured in a humidified 5% CO2 incubator for 2-3 days, providing a 21% atmospheric oxygen level for the cells. After two days, the medium was changed and the cultures were placed in 2% oxygen-level environment provided by a CO2 /Nitrogen gas multigas humidified incubator (Sanyo) for 24hrs. Following the 24hr incubation, the cells were washed with 60mls of 1XPBS and then removed using 40mls 0.25% (w/v) trypsin/EDTA (Gibco). Upon removal, the cell suspension was neutralized with an equal volume of KSFM containing 10% FBS. The cells were then washed by centrifugation 300xg for 10 minutes. After washing, the cells were re-suspended in 20mls of KSFM and transferred to a 50ml conical tube and a sample was collected for cell counting. Once the viable cell count was determined using trypan blue exclusion, 1 million cells were collected for an RNA sample, washed in PBS, and snap frozen in liquid nitrogen. The cells were washed again in PBS and collected by centrifugation at 300xg for 5 minutes. The washed cell pellet was re-suspended in KSFM at a concentration of 37.5 million cells/mi.

Enriching for Specific Bioactive Renal Cells Using Density Step Gradient Separation: Cultured kidney cells, predominantly composed of renal tubular cells but containing small subpopulations of other cell types (collecting duct, glomerular, vascular, and endocrine) were separated into their component subpopulations using a density step gradient made from multiple concentrations w/v of iodixanol (Optiprep). The cultures were placed into a hypoxic environment for up to 24 hours prior to harvest and application to the gradient. A stepped gradient was created by layering four different density mediums on top of each other in a sterile 15mL conical tube, placing the solution with the highest density on the bottom and layering to the least dense solution on the top. Cells were applied to the top of the step gradient and centrifuged, which resulted in segregation of the population into multiple bands based on size and granularity.

Briefly, densities of 7, 11, 13, and 16% Optiprep^{®} (60% w/v Iodixanol) were made using KFSM medium as diluents. For example: for 50mls of 7%(w/v) Optiprep^{®}, 5.83mls of stock 60% (w/v) Iodixanol was added to 44.17mls of KSFM medium and mixed well by inversion. A peristaltic pump (Master Flex L/S) loaded with sterile L/S 16 Tygon tubing connected to sterile capillary tubes was set to a flow rate of 2 ml per minute, and 2 mL of each of the four solutions was loaded into a sterile conical 15 mL tube, beginning with the 16% solution, followed by the 13% solution, the 11% solution, and the 7% solution. Finally, 2 mL of cell suspension containing 75 million cultured rodent kidney cells was loaded a top the step gradient (suspensions having been generated as described above in 'Kidney cell Culture'). Importantly, as the pump was started to deliver the gradient solutions to the tube, care was taken to allow the fluid to flow slowly down the side of the tube at a 45° angle to insure that a proper interface formed between each layer of the gradient. The step gradients, loaded with cells, were then centrifuged at 800 × g for 20 minutes without brake. After centrifugation, the tubes were carefully removed so as not to disturb each interface. Five distinct cell fractions resulted (4 bands and a pellet) (B1 - B4, + Pellet) (see Figure 26, left conical tube). Each fraction was collected using either a sterile disposable bulb pipette or a 5ml pipette and characterized phenotypically and functionally (See Example 10 of Presnell et al. WO/2010/056328). When rodent kidney cell suspensions are subjected to step-gradient fractionation immediately after isolation, the fraction enriched for tubular cells (and containing some cells from the collecting duct) segments to a density between 1.062 - 1.088 g/mL. In contrast, when density gradient separation was performed after ex vivo culture, the fraction enriched for tubular cells (and containing some cells from the collecting duct) segmented to a density between 1.051 - 1.062 g/mL. Similarly, when rodent kidney cell suspensions are subjected to step-gradient fractionation immediately after isolation, the fraction enriched for epo-producing cells, glomerular podocytes, and vascular cells ("B4") segregates at a density between 1.025 - 1.035 g/mL. In contrast, when density gradient separation was performed after *ex* vivo culture, the fraction enriched for epo-producing cells, glomerular podocytes, and vascular cells ("B4") segregated at a density between 1.073 - 1.091 g/mL. Importantly, the post-culture distribution of cells into both the "B2" and the "B4" fractions was enhanced by exposure (for a period of about 1 hour to a period of about 24 hours) of the cultures to a hypoxic culture environment (hypoxia being defined as <21% (atmospheric) oxygen levels prior to harvest and step-gradient procedures (additional details regarding hypoxia-effects on band distribution are provided in Example 7).

Each band was washed by diluting with 3x the volume of KSFM, mixed well, and centrifuged for 5 minutes at 300 x g. Pellets were re-suspended in 2mls of KSFM and viable cells were counted using trypan blue exclusion and a hemacytometer. 1 million cells were collected for an RNA sample, washed in PBS, and snap frozen in liquid nitrogen. The cells from B2 and B4 were used for transplantation studies into uremic and anemic female rats, generated via a two-step 5/6 nephrectomy procedure at Charles River Laboratories. Characteristics of B4 were confirmed by quantitative real-time PCR, including oxygenregulated expression of erythropoietin and vEGF, expression of glomerular markers (nephrin, podocin), and expression of vascular markers (PECAM). Phenotype of the 'B2' fraction was confirmed via expression of E-Cadherin, N-Cadheriii, and Aquaporin-2. See Figures 49a and 49b of Presnell et al. WO/2010/056328.

Thus, use of the step gradient strategy allows not only the enrichment for a rare population of epo-producing cells (B4), but also a means to generate relatively enriched fractions of functional tubular cells (B2) (see Figures 50 & 51 of Presnell et al. WO/2010/056328). The step gradient strategy also allows EPO-producing and tubular cells to be separated from red blood cells, cellular debris, and other potentially undesirable cell types, such as large cell aggregates and certain types of immune cells.

The step gradient procedure may require tuning with regard to specific densities employed to provide good separation of cellular components. The preferred approach to tuning the gradient involves 1) running a continuous density gradient where from a high density at the bottom of the gradient (16-21% Optiprep, for example) to a relatively low density at the top of the gradient (5-10%, for example). Continuous gradients can be prepared with any standard density gradient solution (Ficoll, Percoll, Sucrose, iodixanol) according to standard methods (Axis Shield). Cells of interest are loaded onto the continuous gradient and centrifuged at 800xG for 20 minutes without brake. Cells of similar size and granularity tend to segregate together in the gradients, such that the relative position in the gradient can be measured, and the specific gravity of the solution at that position also measured. Thus, subsequently, a defined step gradient can be derived that focuses isolation of particular cell populations based on their ability to transverse the density gradient under specific conditions. Such optimization may need to be employed when isolating cells from unhealthy vs. healthy tissue, or when isolating specific cells from different species. For example, optimization was conducted on both canine and human renal cell cultures, to insure that the specific 82 and B4 subpopulations that were identified in the rat were isolatable from the other species. The optimal gradient for isolation of rodent 82 and B4 subpopulations consists of (w/v) of 7%, 11%, 13%, and 16% Optiprep. The optimal gradient for isolation of canine B2 and B4 subpopulations consists of (w/v) of 7%, 10%, 11%, and 16% Optiprep. The optimal gradient for isolation of human B2 and B4 subpopulations consists of (w/v) 7%, 9%, 11%, 16%. Thus, the density range for localization of B2 and B4 from cultured rodent, canine, and human renal cells is provided in Table 8.

**Table 8. Species Density Ranges.**

| | Species Density Ranges g/ml | | |
|---|---|---|---|
| Step Gradient Band | Rodent | Canine | Human |
| B2 | 1.045-1.063g/ml | 1.045-1.058g/ml | 1.045-1.052g/ml |
| B4 | 1.073-1.091g/ml | 1.063-1.091g/ml | 1.063-1.091g/ml |

### Example 7 - Low-oxygen culture prior to gradient affects band distribution, composition, and gene expression

To determine the effect of oxygen conditions on distribution and composition of prototypes B2 and B4, neokidney cell preparations from different species were exposed to different oxygen conditions prior to the gradient step. A rodent neokidney augmentation (NKA) cell preparation (RK069) was established using standard procedures for rat cell isolation and culture initiation, as described *supra.* All flasks were cultured for 2-3 days in 21% (atmospheric) oxygen conditions. Media was changed and half of the flasks were then relocated to an oxygen-controlled incubator set to 2% oxygen, while the remaining flasks were kept at the 21% oxygen conditions, for an additional 24 hours. Cells were then harvested from each set of conditions using standard enzymatic harvesting procedures described *supra.* Step gradients were prepared according to standard procedures and the "normoxic" (21% oxygen) and "hypoxic" (2% oxygen) cultures were harvested separately and applied side-by-side to identical step gradients. (Figure 27). While 4 bands and a pellet were generated in both conditions, the distribution of the cells throughout the gradient was different in 21% and 2% oxygen-cultured batches (Table 3). Specifically, the yield of B2 was increased with hypoxia, with a concomitant decrease in B3. Furthermore, the expression of B4-specific genes (such as erythropoietin) was enhanced in the resulting gradient generated from the hypoxic-cultured cells (Figure 73 of Presnell et al. WO/2010/056328).

A canine NKA cell preparation (DK008) was established using standard procedures for dog cell isolation and culture (analogous to rodent isolation and culture procedures), as described *supra.* All flasks were cultured for 4 days in 21% (atmospheric) oxygen conditions, then a subset of flasks were transferred to hypoxia (2%) for 24 hours while a subset of the flasks were maintained at 21%. Subsequently, each set of flasks was harvested and subjected to identical step gradients (Figure 28). Similar to the rat results (Example 6), the hypoxic-cultured dog cells distributed throughout the gradient differently than the atmospheric oxygen-cultured dog cells (Table 9). Again, the yield of B2 was increased with hypoxic exposure prior to gradient, along with a concomitant decrease in distribution into 83.

**Table 9.**

| | **Rat (RK069)** | | **Dog (DK008)** | |
|---|---|---|---|---|
| | **2% O2** | **21% O2** | **2% O2** | **21% O2** |
| **B1** | 0.77% | 0.24% | 1.20% | 0.70% |
| **B2** | 88.50% | 79.90% | 64.80% | 36.70% |
| **B3** | 10.50% | 19.80% | 29.10% | 40.20% |
| **B4** | 0.23% | 0.17% | 4,40% | 21.90% |

The above data show that pre-gradient exposure to hypoxia enhances composition of B2 as well as the distribution of specific specialized cells (erythropoietinproducing cells, vascular cells, and glomerular cells) into B4. Thus, hypoxic culture, followed by density-gradient separation as described *supra*, is an effective way to generate 'B2' and 'B4' cell populations, across species.

### Example 8 - Isolation of tubular/glomerular cells from human kidney

Tubular and glomerular cells were isolated and propagated from normal human kidney tissue by the enzymatic isolation methods described throughout. By the gradient method described above, the tubular cell fraction was enriched ex vivo and after culture. As shown in Figure 68 of Presnell et al. WO/2010/056328 (incorporated herein by reference in its entirety), phenotypic attributes were maintained in isolation and propagation. Tubular cell function, assessed via uptake of labeled albumin, was also retained after repeated passage and cryopreservation. Figure 69 of Presnell et al. WO/2010/056328 (incorporated herein by reference in its entirety) shows that when tubular-enriched and tubular-depleted populations were cultured in 3D dynamic culture, a marked increase in expression of tubular marker, cadherin, was expressed in the tubular-enriched population. This confirms that the enrichment of tubular cells can be maintained beyond the initial enrichment when the cells are cultured in a 3D dynamic environment. The same cultured population of kidney cells described above in Example 7 was subjected to flow cytometric analysis to examine forward scatter and side scatter. The small, less granular EPO-producing cell population was discernable (8.15%) and was separated via positive selection of the small, less granular population using the sorting capability of a flow cytometer (see Figure 70 of Presnell et al. WO/2010/056328 (incorporated herein by reference in its entirety)).

### Example 9 Characterization of an unfractionated mixture of renal cells isolated from an autoimmune glomerulonephritis patient sample

An unfractionated mixture of renal cells was isolated, as described above, from an autoimmune glomerulonephritis patient sample. To determine the unbiased genotypic composition of specific subpopulations of renal cells isolated and expanded from kidney tissue, quantitative real time PCR (qRTPCR) analysis (Brunskill et al., *supra* 2008) was employed to identify differential cell-type-specific and pathway-specific gene expression patterns among the cell subtractions. As shown in Table 6.1 of Ilagan et al. PCT/US2011/036347, HK20 is an autoimmune glomerulonephritis patient sample. As shown in Table 6.2 of Ilagan et al. PCT/US2011/036347, cells generated from HK20 are lacking glomerular cells, as determined by qRTPCR.

### EXAMPLE 11 - Ermchment/Depletion of Viable Kidney Cell Types Using Fluorescent Activated Cell Sorting (FACS)

One or more isolated kidney cells may be enriched, and/or one or more specific kidney cell types may be depleted from isolated primary kidney tissue using fluorescent activated cell sorting (FACS),

**REAGENTS:** 70% ethanol; Wash buffer (PBS ); 50:50 Kidney cell medium (50%DMEM high glucose): 50% Keratinocyte-SFM; Trypan Blue 0.4%; Primary antibodies to target kidney cell population such as CD31 for kidney endothelial cells and Nephrin for kidney glomerular cells. Matched isotype specific fluorescent secondary antibodies; Staining buffer ( 0.05% BSA in PBS).

**PROCEDURE:** Following standard procedures for cleaning the biological safety cabinet (BSC), a single cell suspension of kidney cells from either primary isolation or cultured cells may be obtained from a T500 T/C treated flask and resuspend in kidney cell medium and place on ice. Cell count and viability is then determined using trypan blue exclusion method. For kidney cell enrichment/depletion of, for example, glomerular cells or endothelial cells from a heterogeneous population, between 10 and 50 ×10⁶ live cells with a viability of at least 70% are obtained. The heterogeneous population of kidney cells is then stained with primary antibody specific for target cell type at a starting concentration of 1µg/0.1ml of staining buffer/1 × 10⁶ cells (titer if necessary). Target antibody can be conjugated such as CD31 PE (specific for kidney endothelial cells) or un-conjugated such as Nephrin (specific for kidney glomerular cells).

Cells are then stained for 30 minutes on ice or at 4ºC protected from light. After 30 minutes of incubation, cells are washed by centrifugation at 300xg for 5 min. The pellet is then resuspended in either PBS or staining buffer depending on whether a conjugated isotype specific secondary antibody is required. If cells are labeled with a fluorochrome conjugated primary antibody, cells are resuspended in 2mls of PBS per 10 ×10⁷ cells and proceed to FACS aria or equivalent cell sorter. If cells are not labeled with a fluorochrome conjugated antibody, then cells are labeled with an isotype specific fluorochrome conjugated secondary antibody at a starting concentration of 1µg/0.1ml/1×10⁶ cells.

Cells are then stained for 30 min. on ice or at 4ºC protected from light. After 30 minutes of incubation, cells are washed by centrifugation at 300xg for 5 min. After centrifugation, the pellet is resuspended in PBS at a concentration of 5×10⁶/ml of PBS and then 4mls per 12×75mm is transferred to a sterile tube.

FACs Aria is prepared for live cell sterile sorting per manufacturer's instructions (BD FACs Aria User Manual). The sample tube is loaded into the FACs Aria and PMT voltages are adjusted after acquisition begins. The gates are drawn to select kidney specific cells types using fluorescent intensity using a specific wavelength. Another gate is drawn to select the negative population. Once the desired gates have been drawn to encapsulate the positive target population and the negative population, the cells are sorted using manufacturer's instructions.

The positive target population is collected in one 15ml conical tube and the negative population in another 15 ml conical tube filled with 1 ml of kidney cell medium. After collection, a sample from each tube is analyzed by flow cytometry to determine purity.

Collected cells are washed by centrifugation at 300xg for 5 min. and the pellet is resuspended in kidney cell medium for further analysis and experimentation.

### EXAMPLE 12 -Ennchmerrt/Depletion of Kidney Cell Types Using Magnetic Cell Sorting

One or more isolated kidney cells may be enriched and/or one or more specific kidney cell types may be depleted from isolated primary kidney tissue.

**REAGENTS:** 70% ethanol, Wash buffer (PBS ), 50:50 Kidney cell medium (50%DMEM high glucose): 50% Keratinocyte-SFM, Trypan Blue 0.4%, Running Buffer(PBS, 2mM EDTA,0.5% BSA), Rinsing Buffer (PBS,2mN4 EDTA), Cleaning Solution (70% v/v ethanol), Miltenyi FCR Blocking reagent, Miltenyi microbeads specific for either !gG isotype, target antibody such as CD31(PECAM) or Nephrin, or secondary antibody.

**PROCEDURE:** Following standard procedures for cleaning the biological safety cabinet (BSC), a single cell suspension of kidney cells from either primary isolation or culture is obtained and resuspended in kidney cell medium. Cell count and viability is determined using trypan blue exclusion method. For kidney cell enrichment/depletion of, for example, glomerular cells or endothelial cells from a heterogeneous population, at least 10×10⁶ up to 4 ×10⁹ live cells with a viability of at least 70% is obtained.

The best separation for enrichment/depletion approach is determined based on target cell of interest. For enrichment of a target frequency of less than 10%, for example, glomerular cells using Nephrin antibody, the Miltenyi autoMACS, or equivalent, instrument program POSSELDS (double positive selection in sensitive mode) is used. For depletion of a target frequency of greater than 10%, the Miltenyi autoMACS, or equivalent, instrument program DEPLETES (depletion in sensitive mode) is used.

Live cells are labeled with target specific primary antibody, for example, Nephrin rb polyclonal antibody for glomerular cells, by adding 1µg/10×10⁶ cells/0.1ml of PBS with 0.05% BSA in a 15ml conical centrifuge tube, followed by incubation for 15 minutes at 4ºC.

After labeling, cells are washed to remove unbound primary antibody by adding 1-2ml of buffer per 10 ×10⁷ cells followed by centrifugation at 300xg for 5min. After washing, isotype specific secondary antibody, such as chicken anti-rabbit PE at 1µg/10×10⁵/0.1ml of PBS with 0.05% BSA, is added, followed by incubation for 15 minutes at 4ºC.

After incubation, cells are washed to remove unbound secondary antibody by adding 1-2ml of buffer per 10 ×10⁷ cells followed by centrifugation at 300xg for 5 min. The supernatant is removed, and the cell pellet is resuspended in 60µl of buffer per 10 ×10⁷ total cells followed by addition of 20µl of FCR blocking reagent per 10 ×10⁷ total cells, which is then mixed well.

Add 20 µl of direct MACS microbeads (such as anti-PE microbeads) and mix and then incubate for 15 min at 4ºC.

After incubation, cells are washed by adding 10-20x the labeling volume of buffer and centrifuging the cell suspension at 300xg for 5 min. and resuspending the cell pellet in 500µl -2mls of buffer per 10 ×10⁸ cells.

Per manufacturer's instructions, the autoMACS system is cleaned and primed in preparation for magnetic cell separation using autoMACS. New sterile collection tubes are placed under the outlet ports. The autoMACS cell separation program is chosen. For selection the POSSELDS program is chosen. For depletion the DEPLETES program is chosen.

The labeled cells are inserted at uptake port, then beginning the program.

After cell selection or depletion, samples are collected and placed on ice until use. Purity of the depleted or selected sample is verified by flow cytometry.

### EXAMPLE 13 - Cells with therapeutic potential can be isolated and propagated from normal and chronically-diseased kidney tissue

The objective of the present study was to determine the functional characterization of human NKA cells through high content analysis (HCA). High-content imaging (HCI) provides simultaneous imaging of multiple sub-cellular events using two or more fluorescent probes (multiplexing) across a number of samples. High-content Analysis (HCA) provides simultaneous quantitative measurement of multiple cellular parameters captured in High-Content Images. In brief, unfractionated (UNFX) cultures were generated (Aboushwareb et *a*/*., supra* 2008) and maintained independently from core biopsies taken from five human kidneys with advanced chronic kidney disease (CKO) and three non-CKD human kidneys using standard biopsy procedures. After (2) passages of UNFX *ex vivo,* cells were harvested and subjected to density gradient methods (as in Example 2) to generate subtractions, including subtractions B2, B3, and/or B4.

Human kidney tissues were procured from non-CKD and CKD human donors as summarized in Table 10.1 of Ilagan et al. PCT/US2011/036347. Figure 4 of Ilagan et al. PCT/US2011/036347 shows histopathologic features of the HK17 and HK19 samples. Ex vivo cultures were established from all non-CKD (3/3) and CKD (5/5) kidneys. High content analysis (HCA) of albumin transport in human NKA cells defining regions of interest (ROI) is shown in Figure 5 (HCA of albumin transport in human NKA cells) of Ilagan et al. PCT/US2011/036347. Quantitative comparison of albumin transport in NKA cells derived from non-CKD and CKD kidney is shown in Figure 6 of Ilagan et al. PCT/US2011/036347,

As shown in Figure 6 of Ilagan et al. PCT/US2011/036347, albumin transport is not compromised in CKD-derived NKA cultures. Comparative analysis of marker expression between tubular-enriched B2 and tubular cell-depleted B4 subfractions is shown in Figure 7 (CK8/18/19) of Ilagan et al. PCT/US2011/036347.

Comparative functional analysis of albumin transport between tubular-enriched B2 and tubular cell-depleted B4 subfractions is shown in Figure 8 of Ilagan et al. PCT/US2011/036347. Subtraction B2 is enriched in proximal tubule cells and thus exhibits increased albumin-transport function.

*Albumin uptake*: Culture media of cells grown to confluency in 24-well, collagen IV plates (BD Biocoat ^{™}) was replaced for 18-24 hours with phenol red-free, serumfree, low-glucose. DMEM (pr-/s-/lg DMEM) containing 1X antimycotic/antibiotic and 2mM glutamine. Immediately prior to assay, cells were washed and incubated for 30 minutes with pr-/s-/lg DMEM + 10mM HEPES, 2mM glutamine, 1.8mM CaCl2, and 1mM MgCI2. Cells were exposed to 25µg/mL rhodamine-conjugated bovine albumin (invitrogen) for 30 min, washed with ice cold PBS to stop endocytosis and fixed immediately with 2% paraformaldehyde containing 25 µg/mL Hoechst nuclear dye. For inhibition experiments, 1µM receptorassociated protein (RAP) (Ray Biotech, Inc., Norcross GA) was added 10 minutes prior to albumin addition. Microscopic imaging and analysis was performed with a BD Pathway^{™} 855 High-Content Biolmager (Becton Dickinson) (see Kelley et al. Am J Physiol Renal Physiol. 2010 Nov; 299(5):F1026-39. Epub Sep 8, 2010).

in conclusion, HCA yields cellular level data and can reveal populations dynamics that are undetectable by other assays, *i.e*., gene or protein expression. A quantifiable *ex-vivo* HCA assay for measuring albumin transport (HCA-AT) function can be utilized to characterize human renal tubular cells as components of human NKA prototypes. HCA-AT enabled comparative evaluation of cellular function, showing that albumin transport-competent cells were retained in NKA cultures derived from human CKD kidneys. It was also shown that specific subfractions of NKA cultures, B2 and B4, were distinct in phenotype and function, with B2 representing a tubular cell-enriched fraction with enhanced albumin transport activity. The B2 cell subpopulation from human CKD are phenotypically and functionally analogous to rodent B2 cells that demonstrated efficacy *in* vivo (as shown above).

### Literature References

1. Kelley, R. et al. Intra-renal Transplantation of Bioactive Renal Cells Preserves Renal Functions and Extends Survival in the ZSFI model of Progressive Diabetic Nephropathy. 2011 American Diabetes Association (ADA) Conference. (2011).
2. Kelley, R. et al. Tubular cell-enriched subpopulation of primary renal cells improves survival and augments kidney function in rodent model of chronic kidney disease. Am J Physiol Renal Physiol 299, F1026-1039,
3. Presnell, S.C. et al. (2011) Isolation, Characterization, and Expansion Methods for Defined Primary Renal Cell Populations from Rodent, Canine, and Human Normal and Diseased Kidneys, Tissue Eng Part C Methods 17:261-273.
4. Humphreys, B.D. et of. Repair of injured proximal tubule does not involve specialized progenitors. Proc Natl Acad Sci U S A 108, 9226-9231.
5. Aboushwareb, T. et al. Erythropoietin producing cells for potential cell therapy. World J Urol 26, 295-300 (2008).
6. Takano, Y. et al. Recovery and maintenance of nephrin expression in cultured podocytes and identification of HGF as a repressor of nephrin. Am J Physiol Renal Physiol 292, F1573-1582 (2007).
7. Basu, J. et al, Expansion of the human adipose-derived stromal vascular cell fraction yields a population of smooth muscle-like cells with markedly distinct phenotypic and functional properties relative to mesenchymal stem cells. Tissue Eng Part C Methods 17, 843-860,
8. Basu, J. et al. Organ specific regenerative markers in peri-organ adipose: kidney. Lipids Health Dis 10, 171.
9. Xinaris C, et al. in vivo maturation of functional renal organoids formed from embryonic cell suspensions. J Am Soc Nephrol 23,1-12, (2012).
10. Buzhor et al. Kidney Spheroids Recapitulate Tubular Organoids Leading to Enhanced Tubulogenic Potency of Human Kideny-Derived Cells Tissue Engineering Part A, (2011).
11. Brown, SA et al., impaired renal auto regulatory ability in dogs with reduced renal mass. J Am Soc Nephrol. 5:1768-74 (1995).
12. Robertson, J.L. et al., Long-term renal responses to high dietary protein in dogs with 75% nephrectomy. Kidney Int. 29:511-9 (1986).
13. Urie, B.K. et al., Evaluation of clinical status, renal function, and hematopoietic variables after unilateral nephrectomy in canine kidney donors. J Am Vet Med Assoc. 230:1653-6 (2007).

The following paragraphs describe embodiments of the invention.
1. A method of forming an organoid comprising a heterogeneous renal cell population and a bioactive cell population, said method comprising culturing the heterogeneous renal cell population and a bioactive cell population in a culture system selected from the group consisting of i) 2D culture; ii) 3D culture: COL(I) gel; iii) 3D culture: Matrigel; iv) 3D culture: spinners, followed by COL(I)/Matrigel; and v) 3D culture: COL(IV) gel.
2. The method of paragraph 1 wherein the heterogeneous renal cell population comprises a bioactive renal cell population.
3. The method of paragraph I wherein the heterogeneous renal cell population comprises a B2 cell population comprising an enriched population of tubular cells, and wherein the heterogeneous renal cell population is depleted of a B1 cell population.
4. The method of paragraph 3 wherein the heterogeneous renal cell population is further depleted of a B5 cell population.
5. The method of paragraph 1 wherein the heterogeneous renal cell population comprises a cell population selected from B2, B2/B3, B2/B4, and B2/B3/B4.
6. The method of paragraph 1 wherein the heterogeneous renal cell population comprises erythropoetin (EPO)-producing cells.
7. The method of paragraph 1 wherein the bioactive cell population is an endothelial cell population.
8. The method of paragraph 7 wherein the endothelial cell population is a cell line.
9. The method of paragraph 7 wherein the endothelial cell population comprises HUVEC cells.
10. The method of paragraph 1 wherein the cell populations are selected from xenogeneic, syngeneic, allogeneic, autologous and combinations thereof.
11. The method of paragraph 1 wherein the heterogeneous renal cell population and the bioactive cell population are cultured separately for a first time period, combined and cultured for a second time period.
12. The method of paragraph 11 wherein the second time period is at least 24 hours.
13. The method of paragraph 12 wherein the second time period is 24 hours to 72 hours.
14. The method of paragraph 1 wherein the renal cell population and bioactive cell population are at a ratio of 1:1.
15. The method of paragraph 1 wherein the renal cell population and bioactive cell population are suspended in growth medium,
16. An organoid made according to the method of any one of paragraphs 1 to 15.
17. An organoid comprising a heterogeneous renal cell population and a bioactive cell population.
18. The organoid of paragraph 17 wherein the bioactive cell population is an endothelial cell population.
19. The organoid of paragraph 18 wherein the heterogeneous renal cell population comprises a B2 cell population comprising an enriched population of tubular cells, and wherein the heterogeneous renal cell population is depleted of a B1 cell population.
20. The organoid of paragraph 19 wherein the heterogeneous renal cell population is further depleted of a B5 cell population.
21. The organoid of paragraph 17 wherein the heterogeneous renal cell population comprises a cell population selected from B2/B3, B2/B4, and B2/B3/B4.
22. The organoid of paragraph 17 wherein the heterogeneous renal cell population comprises erythropoetin (EPO)-producing cells.
23. The organoid of paragraph 18 wherein the endothelial cell population is a cell line.
24. The organoid of paragraph 18 wherein the endothelial cell population comprises HUVEC cells.
25. An injectable formulation comprising at least one organoid and a liquid medium.
26. The formulation of paragraph 25, wherein the liquid medium is selected from a cell growth medium, DPBS and combinations thereof.
27. The formulation of paragraph 26 wherein the liquid medium is DPBS.
28. The formulation of paragraph 25, wherein the organoids are suspended in the liquid medium.
23. An injectable formulation comprising organoids and a temperature-sensitive cell-stabilizing biomateriai that maintains
   (i) a substantially solid state at about 8°C or below, and
   (ii) a substantially liquid state at about ambient temperature or above.
30. The formulation of paragraph 29, wherein the organoids comprise bioactive renal cells.
31. The formulation of paragraph 30, wherein the organoids further comprise HUVEC.
32. The formulation of paragraph 29, wherein the bioactive cells are substantially uniformly dispersed throughout the volume of the cell-stabilizing biomaterial.
33. The formulation of paragraph 29, wherein the biomaterial comprises a solid-to-liquid transitional state between about 8°C and about ambient temperature or above.
34. The formulation of paragraph 29, wherein the substantially solid state is a gel state.
35. The formulation of paragraph 29, wherein the cell-stabilizing biomaterial comprises a hydrogel.
36. The formulation of paragraph 35, wherein the hydrogel comprises gelatin.
37. The formulation of paragraph 36, wherein the gelatin is present in the formulation at about 0.5% to about 1% (w/v),
38. The formulation of claim 36, wherein the gelatin is present in the formulation at about 0.75% (w/v).
39. A method of treating kidney disease in a subject in need comprising administering at least one organoid comprising a heterogeneous renal cell population and a bioactive cell population.
40. The method of paragraph 39 wherein the bioactive cell population is an endothelial cell population.
41. The method of paragraph 39 wherein the heterogeneous renal cell population comprises a B2 cell population comprising an enriched population of tubular cells, and wherein the heterogeneous renal cell population is depleted of a B1 cell population.
42. The method of paragraph 41 wherein the heterogeneous renal cell population is further depleted of a B5 cell population.
43. The method of paragraph 39 wherein the heterogeneous renal cell population comprises a cell population selected from B2, B2/B3, B2/B4, and B2/B3/B4.
44. The method of paragraph 39 wherein the heterogeneous renal cell population comprises erythropoetin (EPO)-producing cells.
45. The method of paragraph 40 wherein the endothelial cell population is a cell line.
46. The method of paragraph 40 wherein the endothelial cell population comprises HUVEC cells.
47. A method of treating kidney disease in a subject in need comprising administering an injectable formulation according to any one of paragraphs 25-38.
48. A method according to any one of paragraphs 39-46 wherein the subject is a mammal.
49. The method according to paragraph 48 wherein the mammal is a human.
50. The method according to any one of paragraphs 39-46 wherein the subject has a kidney disease.
51. The method according to any one of paragraphs 39-46 wherein an improvement in any one of the following measures of anemia (Hct, Hgb, RBC), inflammation (WBC), urine concentration (spGrav) and azotemia (BUN) is observed.
52. Use of an organoid in the preparation of a medicament for the treatment of kidney disease.

## Claims

1. An organoid comprising a heterogeneous renal cell population and a non-renal bioactive cell population,
wherein the heterogeneous renal cell population is derived from a starting kidney cell population,
wherein the heterogeneous renal cell population comprises a greater percentage of EPO-producing cells than does the starting kidney cell population,
wherein the starting kidney cell population is a kidney biopsy sample, whole kidney tissue or an *in vitro* culture of cells established from either a kidney biopsy sample or whole kidney tissue, and
wherein the non-renal bioactive cell population is a non-renal endothelial cell population, a non-renal endothelial progenitor cell population, a non-renal mesenchymal stem cell population, or a non-renal adipose-derived progenitor cell population, and
wherein the organoid is cultured as a cluster of cells in media in suspension without attachment to a scaffold.

2. The organoid of claim 1, wherein the heterogeneous renal cell population further comprises a greater percentage of glomerular and vascular cells than does the starting population.

3. The organoid of claim 1 or 2, wherein the non-renal bioactive cell population is the non-renal endothelial cell population.

4. The organoid of claim 3, wherein the non-renal endothelial cell population is a cell line.

5. The organoid of claim 3, wherein the non-renal endothelial cell population comprises human umbilical vein endothelial cells (HUVECs).

6. The organoid of claim 1 or 2, wherein the non-renal bioactive cell population is the non-renal endothelial progenitor cell population, the non-renal mesenchymal stem cell population or the non-renal adipose-derived progenitor cell population.

7. A method of forming an organoid comprising:
culturing a heterogeneous renal cell population and a non-renal bioactive cell population in a three-dimensional (3D) culture system until the organoid forms,
wherein the 3D culture system comprises a spinner flask and lacks an exogenous scaffold to which cells of the heterogeneous renal cell population and the non-renal bioactive cell population attach,
wherein the heterogeneous renal cell population is derived from a starting kidney cell population,
wherein the starting kidney cell population is a kidney biopsy sample, whole kidney tissue or an *in vitro* culture of cells established from either a kidney biopsy sample or whole kidney tissue,
wherein the heterogeneous renal cell population comprises a greater percentage of EPO-producing cells than does the starting kidney cell population; and
wherein the non-renal bioactive cell population is a non-renal endothelial cell population, a non-renal endothelial progenitor cell population, a non-renal mesenchymal stem cell population, or a non-renal adipose-derived progenitor cell population.

8. The method of claim 7, wherein the heterogeneous renal cell population further comprises a greater percentage of glomerular and vascular cells than does the starting population.

9. The method of claim 7 or 8, wherein the non-renal bioactive cell population is the non-renal endothelial cell population.

10. The method of claim 9, wherein the non-renal endothelial cell population comprises HUVECs.

11. The method of claim 9, wherein the non-renal endothelial cell population is a cell line.

12. The method of claim 7 or 8, wherein the non-renal bioactive cell population is the non-renal endothelial progenitor cell population, the non-renal mesenchymal stem cell population or the non-renal adipose-derived cell population.

13. An injectable formulation comprising at least one organoid of any of claims 1-6 and a liquid medium, optionally,
wherein the liquid medium comprises a cell growth medium, Dulbecco's Phosphate-Buffered Saline or a combination thereof.

14. An injectable formulation comprising at least one organoid of any of claims 1-6 and a temperature-sensitive cell-stabilizing biomaterial that maintains:
(i) a substantially solid state at about 8°C or below, and
(ii) a substantially liquid state at about ambient temperature or above, optionally,
wherein the biomaterial comprises gelatin, optionally,
wherein the gelatin is present in the formulation at about 0.5% to about 1% (w/v).

15. The organoid of any of claims 1-6, or the injectable formulation of claim 13 or 14, for use in a method of treating kidney disease in a subject in need.
